(19) Europäisches Patentamt
European Patent Office
Office européen des brevets

(11) **EP 3 786 733 B1**

(12) **FASCICULE DE BREVET EUROPEEN**

(45) Date de publication et mention
de la délivrance du brevet:
**12.04.2023 Bulletin 2023/15**

(21) Numéro de dépôt: **19306046.4**

(22) Date de dépôt: **29.08.2019**

(51) Classification Internationale des Brevets (IPC):
**G05B 17/02** (2006.01)   **G16C 20/10** (2019.01)

(52) Classification Coopérative des Brevets (CPC):
**G05B 17/02; G16C 20/10**

(54) **SYSTÈME ET PROCÉDÉ POUR LA SIMULATION D'UN PROCÉDÉ CHIMIQUE OU BIOCHIMIQUE**

SYSTEM UND VERFAHREN FÜR DIE SIMULATION EINES CHEMISCHEN ODER
BIOCHEMISCHEN VERFAHRENS

SYSTEM AND METHOD FOR SIMULATING A CHEMICAL OR BIOCHEMICAL PROCESS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(43) Date de publication de la demande:
**03.03.2021 Bulletin 2021/09**

(73) Titulaire: **YF1
54000 Nancy (FR)**

(72) Inventeurs:
• **NICOUD, Roger-Marc
54690 Lay-St-Christophe (FR)**
• **BERGER, Etienne
54000 Nancy (FR)**

(74) Mandataire: **August Debouzy
7, rue de Téhéran
75008 Paris (FR)**

(56) Documents cités:
**US-A1- 2005 267 723     US-A1- 2007 005 317
US-B1- 6 983 227**

• **Tom Marlin: "Chapter 3: Mathematical Modelling
Principles", Process Control, 1 janvier 1999
(1999-01-01), pages 49-95, XP055534861, Extrait
de l'Internet:
URL:http://pc-textbook.mcmaster.ca/Marlin-
Ch03.pdf [extrait le 2018-12-14]**
• **BELAUD JEAN-PIERRE ET AL: "Collaborative
simulation and scientific big data analysis:
Illustration for sustainability in natural hazards
management and chemical process
engineering", COMPUTERS IN INDUSTRY,
ELSEVIER, AMSTERDAM, NL, vol. 65, no. 3, 7
février 2014 (2014-02-07), pages 521-535,
XP028630306, ISSN: 0166-3615, DOI:
10.1016/J.COMPIND.2014.01.009**

**Description**

DOMAINE TECHNIQUE

La présente invention concerne le domaine de la modélisation et de la simulation en génie des procédés. En particulier, la présente invention porte sur un système et un procédé de simulation par ordinateur d'un procédé chimique ou biochimique.

ETAT DE LA TECHNIQUE

Les industriels de la chimie fine ou des biotechnologies doivent fréquemment estimer ou comparer des procédés de production impliquant plusieurs opérations et divers équipements à partir d'une quantité d'informations très limitée. C'est tout particulièrement le cas dans les premiers stades de développement. Notons que dans ces industries, lorsqu'un procédé de production doit être imaginé, il arrive fréquemment que les chances de mise sur le marché de la molécule visée soient très faibles, par exemple, pour des raisons d'échecs d'essais cliniques. Il existe donc de très nombreuses estimations technico-économiques à effectuer sur de nombreuses molécules qui ne seront produites qu'en faibles quantités et pendant une période de temps limitée.

Lorsque les industriels souhaitent obtenir simplement une simulation rapide d'un procédé chimique ou biochimique en l'absence de connaissances de l'état interne des équipements mettant en oeuvre le procédé, les managers et ingénieurs estiment les performances des différentes opérations à l'aide de connaissances empiriques ou même simplement d'intuition. Ces connaissances sont du type : la teneur en composé A en sortie du réacteur est de 50% sans que cette teneur soit reliée à une connaissance des conditions internes du réacteur ou bien le taux de récupération du composé A dans le séparateur est de 95% sans que cette teneur soit reliée à une connaissance des conditions internes du séparateur. Ces informations figées permettent de relier les quantités de sortie (aussi appelées quantités finales en régime transitoire ; par la suite on se référera indifféremment aux termes quantités finales ou quantités de sortie) aux quantités d'entrée (aussi appelées quantités initiales en régime transitoire ; par la suite on se référera indifféremment aux termes quantités initiales ou quantités d'entrée). Cette méthode ne fait pas appel à des bilans de matière ou de chaleur et ne prend donc pas en compte les états internes des équipements. Elle est donc incapable de prévoir une dynamique. A partir de la connaissance des quantités d'entrée du procédé il est possible de calculer de proche en proche, pour chaque opération, les quantités de sortie. On peut donc connaître à l'aide d'un simple calculateur, comme par exemple un tableur Excel, les quantités de sortie obtenues à l'issue du procédé.

Cette méthode présente l'avantage de la simplicité et de la rapidité de calcul. Cependant, elle présente plusieurs inconvénients :

- le nombre de scenarii envisageables est limité aux connaissances expérimentales des opérateurs, les optimisations et comparaisons fines entre les procédés ne sont donc pas possibles ;

- lorsque de nombreuses opérations se déroulent en série au cours du procédé, la simulation devient particulièrement complexe et imprécise. Cette complexité est accentuée en cas de boucle de recyclage de composés au cours du procédé ;

- les régimes transitoires ne sont pas pris en compte. Cette méthode est limitée aux systèmes continus ou aux systèmes discontinus par lots dans lesquels on ne prend pas en compte les régimes transitoires. Or, bien souvent les quantités produites par les industriels de la chimie fine ou des biotechnologies ne permettent pas de recourir à des productions en continu (contrairement aux industries pétrochimiques).

Lorsque les industriels souhaitent connaître avec plus de précision les quantités finales et les performances d'un procédé il est nécessaire d'avoir recours à des méthodes éprouvées du génie des procédés, basées sur des modélisations détaillées, tels que Honeywell UNISIM®, Aspen HYSYS®. Ces modèles nécessitent la connaissance d'un très grand nombre de paramètres physico-chimiques en particulier thermodynamiques qui permettent de simuler le comportement et les performances du système en fonction des conditions internes. Ces modèles prennent en compte les bilans de matière et de chaleur et déterminent l'évolution de variables d'état interne aux équipements. Cette méthode bien que plus précise est très peu utilisée par les industriels de la chimie fine ou des biotechnologies car elle nécessite la prise en compte d'un grand nombre de variables d'état interne et le recours à des méthodes numériques très complexes pour un non-spécialiste. Le temps et les ressources nécessaires à la détermination des paramètres physico-chimiques et à la mise au point de l'outil de simulation sont trop importants pour un procédé qui pourrait par la suite n'être utilisé que pour des quantités très réduites.

US 2005/267723 A1 décrit un système de modélisation de procédés qui impliquent la manipulation d'espèces chimiques telles que par des réactions chimiques, des transferts de masse, des transferts de phase, ou des flux.

Aucune des approches existantes à ce jour n'est satisfaisante. Elles sont soit limitées à des scenarii particulièrement simplistes, soit trop complexes et requièrent la détermination d'un trop grand nombre de paramètres.

Il existe donc un besoin pour un système et un procédé pour la simulation par ordinateur d'un procédé chimique ou biochimique permettant de simuler des scénarii complexes, incluant plusieurs opérations, dans un temps raisonnable tout en limitant le nombre de paramètres physico-chimiques requis pour effectuer la simulation. Il existe en particulier un besoin pour un outil industriel, aujourd'hui inexistant permettant une coopération entre divers intervenants dans le dimensionnement et la réalisation d'installations chimiques ou biochimiques complexes.

La présente invention s'inscrit dans ce cadre.

## RESUME DE L'INVENTION

Les modes de réalisation de l'invention offrent un outil industriel d'ingénierie des procédés de chimie fine. Une caractéristique technique fonctionnelle des modes de réalisation réside dans la simulation de procédés pour leur mise en oeuvre ultérieure dans des installations de production de produits chimiques ou biochimiques.

Les modes de réalisation permettent de prédire, de façon concrète le comportement de procédés chimiques ou biochimiques pour faire un certain nombre d'estimations, notamment sur leur faisabilité industrielle. Les modes de réalisation de l'invention permettent ainsi d'orienter le développement de procédés chimiques ou biochimiques avec une précision suffisante, et dans des temps et des coûts raisonnables, pour permettre d'estimer les chances de réussite de leur mise en oeuvre industrielle et ce, avant même la mise en place d'une installation et la mise en fonctionnement de cette installation.

Les modes de réalisation de l'invention offrent ainsi l'outil industriel manquant aujourd'hui dans le développement des installations chimiques ou biochimiques. Ils permettent de déterminer très en amont, la faisabilité industrielle ou les éventuelles difficultés de mise en oeuvre de procédé ou d'installations chimiques ou biochimiques.

Une mise en oeuvre purement intellectuelle d'une simulation de procédés chimiques ou biochimiques n'est pas possible. C'est en particulier le cas des simulations pour des besoins industriels. Il est en pratique impossible de réaliser les calculs nécessaires pour arriver à des résultats suffisamment précis et significatifs permettant de prendre des décisions industrielles comme par exemple la mise en place d'installations chimiques. En outre, la mise en oeuvre purement intellectuelle de la simulation prendrait un temps prohibitif, ne particulier pour caler les paramètres du modèle.

Or, sans une simulation assistée par ordinateur, il est impossible de tester de façon prédictive ou de faire un choix parmi une pluralité de projets de procédés chimiques ou biochimiques ceux qui offrent les meilleurs performances, et ce, dans un temps industriellement raisonnable.

Il n'existe aucune méthode purement intellectuelle, mathématique ou encore théorique qui permette de prédire de manière exhaustive et rapide le comportement d'un procédé de chimie fine.

Un but de l'invention est de permettre aux industries de la chimie fine et des biotechnologies l'utilisation de simulateurs pertinents en fournissant un outil utilisable par un non expert de la simulation, qui autorise la prise en compte d'un projet de production avec des connaissances de physico-chimie initialement quasi inexistantes.

Cet outil doit avoir la capacité d'aider l'utilisateur à identifier les points délicats ou limitants du procédé en évaluation afin de déterminer les connaissances à acquérir ou à utiliser afin d'atteindre un objectif donné, à affiner ces prévisions au fur à et mesure de la disposition des nouvelles connaissances. Cet outil devra de plus être fiable, facile à utiliser, et permettre une précision d'estimation des performances technico-économiques du procédé étudié qui soit adaptée au problème posé et qui pourra évoluer en fonction des besoins et de l'évolution du projet industriel.

Les modes de réalisation de l'invention reposent sur une simulation tenant compte des caractéristiques physico-chimiques des réactifs et des intermédiaires utilisés dans le procédé ou l'installation chimique ou biochimique simulée.

A cette fin, selon un premier aspect, l'invention concerne un système selon la revendication 1.

## DÉFINITIONS

Par « chimie fine » également appelée « chimie de spécialité » on entend dans le présent document une branche de l'industrie chimique qui synthétise des produits spécifiques, dans de faibles volumes de production, mais à haute valeur ajoutée, et répondant à des contraintes techniques, par exemple de pureté, élevées.

Par « biotechnologie » on entend des technologies de production de molécules par fermentation, cultures de cellules, extraction du milieu naturel.

Par « équation algébrique » on entend une équation ayant pour inconnue une ou plusieurs variables réelles.

Par « équation différentielle » on entend une équation ayant pour inconnue une ou plusieurs fonctions ; elle se présente sous la forme d'une relation entre ces fonctions inconnues et leurs dérivées successives.

Par « équation explicite » on entend une équation entre différentes variables où une variable est explicitée en fonction des autres.

Par « équation implicite » on entend une équation entre différentes variables où une variable n'est pas explicitée en fonction des autres.

Par « variable d'état interne », on entend une variable chimique qui décrit ce qui se passe à l'intérieur d'un équipement donné, par exemple la température, la pression, la concentration...

Par « variable d'état d'entrée » ou « variable d'état de sortie », on entend une variable chimique qui décrit ce qui rentre ou sort, respectivement, d'un équipement donné, par exemple la température, la pression, la concentration...

Par « pseudo variable d'état interne » on entend une variable artificielle permettant de transformer des « équations algébriques explicites » simples reliant une sortie à une entrée dans un formalisme mathématique identique à celui des modèles prédictifs utilisant des « équations algébro-différentielles ».

Par « modèle prédictif » on entend l'association d'un certain nombre d'équations algébriques et différentielles basées sur des concepts de chimie et ou de physique dont la résolution permet la détermination des variables d'état interne d'au moins une opération effectuée dans au moins un équipement.

Par « opération » on entend une transformation permettant de passer d'un état d'entrée à un état de sortie (ou d'un état initial à un état final). Généralement un procédé chimique ou biochimique comprend une pluralité d'opérations.

DESCRIPTION DES FIGURES

La figure 1 illustre un procédé chimique mettant en oeuvre plusieurs opérations 01, 02, 03, 04 et plusieurs équipements E1, E2, E3, E4.

La figure 2 illustre de façon schématique le système conformément à un mode de réalisation de l'invention.

La figure 3 est un diagramme illustrant des étapes pour effectuer la simulation conformément à un mode de réalisation de l'invention.

La figure 4 représente un diagramme bloc représentant un dispositif pour implémenter un ou plusieurs modes de réalisation de l'invention de l'invention.

La figure 5 représente un schéma de réaction comprenant un réacteur avec une entrée In et deux sorties Out1, Out2.

La figure 6 illustre schématiquement un système 600 selon des modes de réalisation,

La figure 7 illustre un mode de conception de l'art antérieur,

La figure 8 illustre les traitements dans un système selon l'invention,

La figure 9 est un diagramme d'opération du procédé traditionnel de purification de la DVL,

La figure 10 est un diagramme d'opérations du procédé de purification de la DVL avec recyclage du solvant d'extraction,

La figure 11 est un diagramme d'opérations du procédé de purification de la DVL par neutralisation thermique du peroxyde,

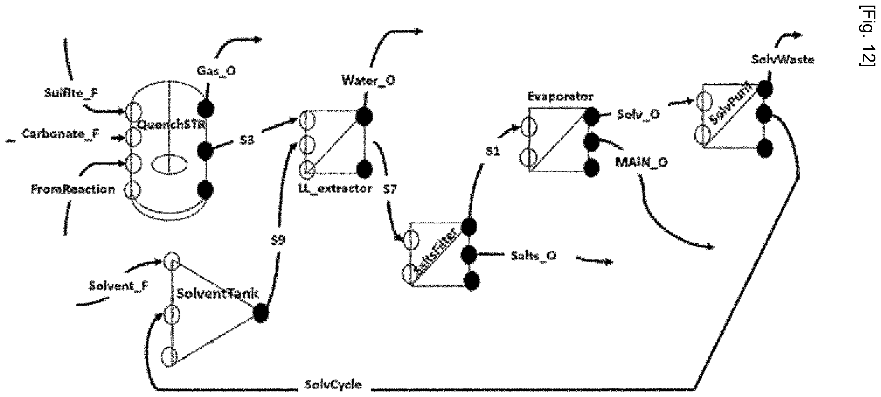

La figure 12 est un diagramme d'équipements du procédé de purification de la DVL avec recyclage du solvant d'extraction,

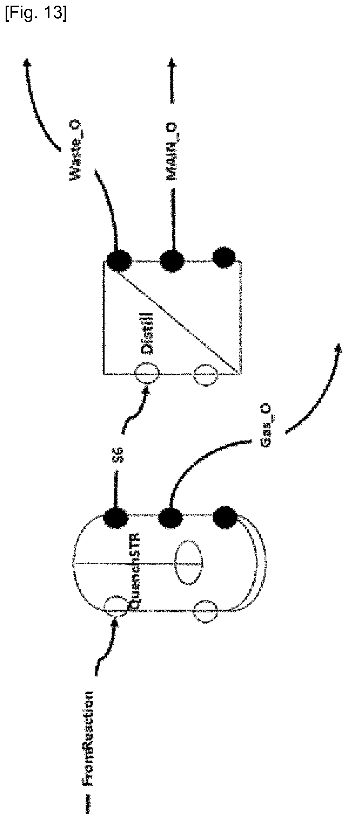

La figure 13 est un diagramme d'équipements du procédé de purification de la DVL par neutralisation thermique du peroxyde,

La figure 14 est un diagramme d'opérations du procédé de production sertraline-tetralone sans racémisation,

La figure 15 est un diagramme d'opérations du procédé de production sertraline-tetralone avec racémisation,

La figure 16 est un diagramme d'opérations du procédé de production sertraline-tetralone avec racémisation et recyclage de l'acétonitrile de racémisation,

La figure 17 est un diagramme d'équipements du procédé de production sertraline-tetralone sans racémisation,

La figure 18 est un diagramme d'équipements du procédé de production sertraline-tetralone avec racémisation et recyclage de l'acétonitrile de racémisation.

DESCRIPTION DETAILLÉE

Un procédé chimique peut être découpé en une pluralité d'opérations réalisées dans une pluralité d'équipements. Le nombre d'opérations peut être inférieur, supérieur ou égal au nombre d'équipements. En effet, le nombre d'opérations peut être supérieur au nombre d'équipements si plusieurs opérations sont effectuées dans le même équipement tandis que le nombre d'équipements peut être supérieur au nombre d'opérations si une opération nécessite plusieurs équipements.

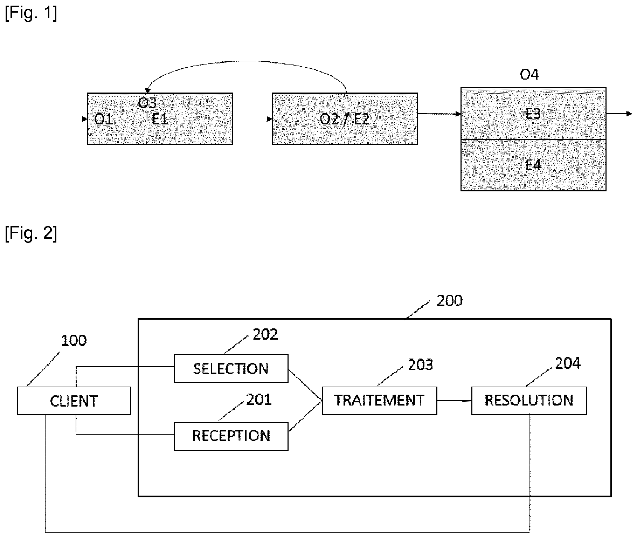

La **figure 1** illustre schématiquement ces différents cas de figures avec diverses opérations O1 à O4 et divers équipements E1 à E4 pour mettre en oeuvre ces opérations. Par exemple les opérations peuvent consister en des mélanges, des séparations et des réactions divers. Par exemple les équipements peuvent consister en des réacteurs, des mélangeurs des extracteurs, des filtres des évaporateurs etc. Dans ce schéma, une matière première est reçue par l'équipement E1 pour réaliser une opération O1. Le produit issu de l'équipement 1 (qui n'est pas seulement le résultat de l'opération O1 comme on le voit ci-après), est fourni à l'équipement E2 pour réaliser l'opération 02. Une boucle de rétroaction existe entre les équipements E2 et E1. Ainsi, le produit issu de l'équipement E2 est fourni à la fois à l'équipement E1 et à l'équipement E3. Une opération supplémentaire 03 est donc aussi réalisée dans l'équipement E1. En raison de la boucle de retour, les opérations O1 et O3 sont donc réalisées au sein du même équipement E1. Le produit issu de l'équipement E2 est aussi fourni à l'équipement E4. Ainsi, l'opération 04 est réalisée à l'aide de deux équipements E3 et E4. Par exemple, E3 peut être un réacteur et l'équipement E4 un échangeur de chaleur).

Le procédé chimique et l'installation correspondante illustrées par la figure 1, pour être validées industriellement, en vue par exemple d'une mise en production du produit issu du procédé ou le lancement de la construction d'une installation nécessite deux étapes principales. Premièrement, il convient de fournir au concepteur et en fait, en réalité à toute une équipe de conception regroupant des chimistes et aussi des non chimiste (par exemple pour les aspects financier qui entrent évidemment en ligne de compte dans une conception industrielle), un outil permettant de définir ce procédé et cette installation. Deuxièmement, il convient de lui (leur) fournir un moyen de prédiction, c'est-à-dire de simulation, de divers critères lui (leur) permettant de juger de la faisabilité industrielle du projet. Une telle prédiction ou simulation comporte aussi la possibilité de comparer différents procédés ou installation entre elles en vue de faire un choix optimal.

L'outil permettant tout cela doit être efficace en termes de précision mais aussi en termes de simplicité d'utilisation et de temps de calcul. Ainsi, la précision 'est pas toujours le seul paramètre pertinent dans la conception car dans les tous premiers temps de développement, il convient tout d'abord de s'assurer qu'il est pertinent de se lancer dans une étude complète de faisabilité. Ainsi, dans le premiers temps, des estimations grossières peuvent être admises, si elles sont suffisantes pour une prise de décision sur l'opportunité de continuer.

Dans un premier temps, on décrit dans la suite, le mode de simulation utilisé selon des modes de réalisation. Dans un second temps, on décrit un système global, permettant de mettre en oeuvre ce mode de simulation. Dans un troisième temps, des exemples d'utilisation de ce système et un exemple de simulation sont décrits.

**Les modes de simulation**

Une opération (i) et un équipement (j) permettent de passer d'un état d'entrée ( $Y_e^{i/j}$ ) à un état de sortie ( $Y_s^{i/j}$ ). Les variables d'état d'entrée et d'état de sortie sont des vecteurs représentant des grandeurs chimiques en entrée et en sortie d'une opération et d'un équipement d'un procédé chimique ou biochimique.

Lorsqu'une simulation simple et rapide d'une opération et d'un équipement est souhaitée, l'opération et l'équipement sont décrits par des équations algébriques explicites reliant les états d'entrée ( $Y_e^{i/j}$ ) et de sortie ( $Y_s^{i/j}$ ) pour l'opération (i) et de l'équipement (j) : $Y_s^{i/j} = G_e\left(Y_e^{i/j}\right)$ . Il n'y a aucune ambition prédictive dans cette approche.

Lorsqu'une simulation plus complète et précise est souhaitée, il est nécessaire de déterminer ce qui se passe à l'intérieur de l'équipement, et donc de déterminer un vecteur de variables d'état interne ( $X^{i/j}$ ) pour une opération (i) et un équipement (j). Les variables d'état interne sont des vecteurs représentant des grandeurs chimiques internes d'une opération et d'un équipement d'un procédé chimique ou biochimique.

En chimie fine et/ou biotechnologie, les équipements fonctionnent généralement en régime transitoire, les variables d'état interne sont donc le plus souvent des solutions de systèmes d'équations algébriques et différentielles (système algébro-différentiel) qui représentent des bilans de matière, de chaleur ou des équilibres thermodynamiques par exemple.

Dans le cas général, l'opération et l'équipement sont décrits par un système d'équations différentielles faisant intervenir les variables d'état interne. Ces équations relient le vecteur de variables d'état interne ($X^{i/j}$) au vecteur de variables d'état d'entrée ( $Y_e^{i/j}$ ) pour l'opération (i) et l'équipement (j) :

$$P_{RT}^E \left(Y_e^{i/j}, t, X^{i/j}, \frac{dX^{i/j}}{dT}\right)=0.$$

Le vecteur de variables d'état de sortie ( $Y_s^{i/j}$ ) qui peut dépendre du temps est alors déterminé explicitement à partir de la connaissance des variables d'état interne : $Y_s^{i/j}(t) = P_{RT}^E (t, X^{i/j})$ dépendant lui-même du vecteur des variables d'état d'entrée Notons qu'en régime permanent l'état final du vecteur des variables d'état de sortie s'obtient immédiatement par : $Y_s^{i/j}(t = \infty) = P_{RT}^E ( t = \infty, X^{i/j})$ où $t_\infty$ représente le temps de fin de l'opération dans un système discontinu ou un temps suffisamment grand dans un système continu.

Les équations décrivant les opérations et les équipements d'un procédé chimique ou biochimique doivent être résolues de façon couplée en prenant en compte les connexions entre toutes les opérations et tous les équipements au cours de l'ensemble du procédé. Dans le cas d'un système d'équations faisant intervenir les variables d'état interne, cette résolution peut être particulièrement longue en raison de problèmes de convergence mais surtout nécessite la connaissance de paramètres physico-chimiques dont la détermination peut-être longue et difficile.

Deux modes de simulation des opérations/équipements sont ainsi disponibles. L'un assez trivial repose sur de simples relations algébriques explicites entre variables d'état de sortie et d'entrée. L'autre beaucoup plus précis et prédictif impose la résolution d'équation algébro différentielles complexes et la connaissance de nombreux paramètres physico-chimiques. Il est donc souhaitable de pouvoir passer d'une approche détaillée à l'aide d'équations faisant intervenir les variables d'état interne à une approche simplifiée à l'aide d'équations algébriques explicites sans intervention des variables d'état interne pour différentes opérations au sein d'un même procédé. Il serait ainsi possible de gagner un temps précieux en choisissant de simuler au sein d'un procédé chimique ou biochimique une opération simple ou d'influence secondaire à l'aide d'une équation algébrique explicite et une opération plus complexe ou bien critique à l'aide d'un système algébro-différentiel faisant intervenir les variables d'état interne. Cependant, de par la structure mathématique des équations, il n'est pas possible d'intégrer ces deux types d'équations dans un même système de résolution général.

Pour permettre l'intégration de ces deux types d'équations dans un seul système de résolution, les inventeurs ont eu l'idée de remplacer les équations algébriques explicites par des équations algébro-différentielles faisant intervenir des pseudo-variables d'état interne. Les équations algébro-différentielles ainsi créées sont affectées d'une constante de temps très faible devant le temps caractéristique de l'opération. On notera que l'homme du métier connait le temps caractéristique de chaque opération du procédé et saura choisir une constante de temps faible par rapport à ce temps caractéristique (par exemple une constante de temps de l'ordre de la seconde). En choisissant une constante de temps faible, pour un temps bien supérieur à cette constante de temps, le terme différentiel s'annule en régime permanent et il ne reste à s'assurer que les autres termes de l'équation différentielle convergent vers les conditions de l'équation algébrique explicite.

Grâce à cette transformation, les équations algébriques explicites peuvent être intégrées dans le système de simulation et l'utilisateur peut choisir entre une modélisation fine ou non de chaque opération. Ainsi, alors que passer d'un système algébrique à un système algébro-différentiel peut sembler être une complexification de la simulation, cela permet en fait d'apporter une souplesse dans la simulation sans pénaliser les performances de calcul. L'utilisateur peut opter pour l'une ou l'autre des modélisations, en utilisant un même module de résolution d'équations.

A titre d'illustration, l'équation algébrique explicite $Y_s^i = \alpha Y_e^i$ peut être remplacée par l'équation différentielle $\Theta \frac{d\tilde{X}^i}{dT} - \alpha Y_e^i + \tilde{X}^i = 0$ , avec comme condition initiale $\tilde{X}^i(t{=}0) = 0$, où $\Theta$ est une constante de temps et $\tilde{X}^i$ une pseudo-variable d'état interne. Lorsque t est bien supérieur à la constante de temps, la solution $\tilde{X}^i(t)$ est constante, sa dérivée est donc nulle, et par conséquent $\alpha Y_e^i = \tilde{X}^i(\text{t>> } \Theta)$. Pour un temps suffisamment long par rapport à la constante de temps, on retrouve donc la solution de l'équation algébrique explicite ; il suffit pour cela de poser :

$$Y_s^i = \tilde{X}^i(\text{t>> }_\Theta).$$

L'opérateur peut donc choisir de décrire une opération à l'aide d'un système d'équations faisant intervenir les variables d'état interne ou bien une équation algébrique explicite. Si au moins une équation algébrique explicite est sélectionnée pour au moins une opération du procédé, le système selon l'invention remplace cette équation algébrique explicite en équation algébro-différentielle afin de pouvoir l'intégrer dans le système d'équation de l'ensemble du procédé.

La **figure 2** illustre le un schéma-bloc d'un module de simulation selon un mode de réalisation de l'invention. Dans le contexte général de la mise en oeuvre du système, un dispositif client 100 se connecte au système de simulation 200 selon l'invention. Le module de simulation 200 selon l'invention comprend un module de réception 201, optionnellement un module de sélection 202, un module de traitement 203 et un module de résolution d'équations algébro-différentielles 204.

Le dispositif client 100 se connecte au module de réception 201 afin de communiquer au module de réception 201 une équation algébrique explicite représentant une opération chimique ou biochimique et reliant un vecteur de variables d'état d'entrée représentant des grandeurs chimiques initiales de ladite opération à un vecteur de variables d'état de sortie représentant des grandeurs chimiques finales de ladite opération.

Le module de réception 201 est connecté au module de traitement 203 afin de recevoir l'équation algébrique explicite représentant une opération chimique et biochimique et de créer une équation algébro-différentielle de sorte à ce que la solution en régime permanent de l'équation algébro-différentielle ainsi créée converge vers ledit vecteur de variables d'état de sortie selon l'équation algébrique explicite.

Le module de traitement 203 est connecté au module de résolution d'équation algébro-différentielle 204. Lorsque le module de traitement 203 a effectué la création de l'équation algébro-différentielle, le module de résolution 204 résout l'équation afin d'obtenir le vecteur de variables d'état de sortie de l'opération.

Le module de résolution d'équation algébro-différentielle 204 est connecté au dispositif client 100 afin de retourner le vecteur de variables d'état de sortie de l'opération.

Selon un mode de réalisation, le dispositif client 100 communique au module de réception 201 une équation algébrique explicite de la première opération et une équation algébro-différentielle de la première opération. Le dispositif client 100 est connecté au module de sélection 202 de sorte sélectionner une simulation par l'intermédiaire de l'équation algébrique explicite ou une simulation par l'intermédiaire de l'équation algébro-différentielle. En fonction du mode sélectionné, le module de traitement créé une équation algébro-différentielle convergeant vers ledit vecteur de variables d'état de sortie selon l'équation algébrique explicite ou bien utilise l'équation algébro-différentielle existante.

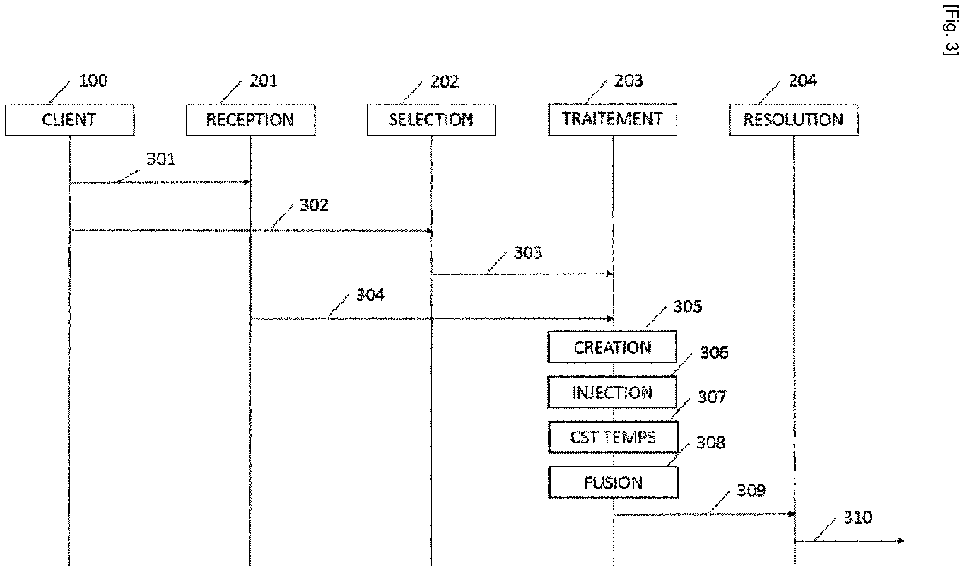

La **figure 3** illustre les étapes pour effectuer la simulation conformément à des modes de réalisation.

Le module client 100 communique avec le module de réception 201 à l'étape 301. Au cours de cette étape 301, le module client communique au module de réception une équation algébrique explicite représentant une première opération chimique ou biochimique et reliant un premier vecteur de variables d'état d'entrée représentant des grandeurs chimiques initiales de ladite première opération à un premier vecteur de variables d'état de sortie représentant des grandeurs chimiques finales de ladite première opération. Selon un mode de réalisation, au cours de cette étape 301, le dispositif client peut également communiquer une équation algébro-différentielle représentant ladite première opération chimique ou biochimique et reliant un premier vecteur de variables d'état d'entrée ; et un premier vecteur de variables d'état interne de ladite première opération en tant qu'inconnue de l'équation. Selon un mode de réalisation, au cours de cette étape 301, le dispositif client peut également communiquer une équation algébro-différentielle représentant une deuxième opération chimique ou biochimique et reliant un deuxième vecteur de variables d'état d'entrée ; et un deuxième vecteur de variables d'état interne de ladite première opération en tant qu'inconnue de l'équation.

Selon un mode de réalisation, le module client 100 communique avec le module de réception à l'étape 302. Lors de cette étape 302, le dispositif client sélectionne un mode de simulation. Le mode de simulation comprend soit la simulation de l'opération chimique par l'intermédiaire de l'équation algébro-différentielle soit par l'intermédiaire de l'équation algébrique explicite.

Lors de l'étape 303 le module de sélection 202 communique au module de traitement le mode de simulation sélectionné et le module de réception transmet 304 au module de traitement l'équation en fonction du mode de simulation sélectionné.

Si une simulation à partir d'une équation algébrique explicite est sélectionnée pour la première opération, le module de traitement effectue l'étape de création 305 d'une première équation algébro-différentielle reliant un premier vecteur de variables d'état d'entrée ; et un premier vecteur de variables d'état interne de ladite première opération en tant qu'inconnue de l'équation. Par la suite, le module de traitement injecte 306 dans la première équation algébro-différentielle l'expression du vecteur de variables d'état de sortie de ladite première opération selon ladite première équation algébrique explicite en tant que vecteur de pseudo-variables d'état interne de ladite première opération, la solution en régime permanent de ladite équation algébro-différentielle convergeant ainsi vers ledit premier vecteur de variables d'état de sortie selon la première équation algébrique explicite. Le module de traitement fixe ensuite à l'étape 307 la constante de temps. Cette constante de temps est inférieure au temps caractéristique de la première opération. Ce temps caractéristique peut être fourni par l'utilisateur via le module client 100 et le module de réception 201 au module de traitement 203.

Le module de traitement transmet ensuite l'équation algébro-différentielle ainsi obtenue au module de résolution pour la résolution de l'équation 309 afin d'obtenir le vecteur de variables d'état de sortie de la première opération.

Selon un mode de réalisation, le module de résolution transmet ce vecteur de variables d'état de sortie de la première opération au dispositif client au cours d'une étape 310.

Selon un mode de réalisation où le procédé comprend deux opérations dont une deuxième opération modélisée dès le départ par une équation algébro-différentielle, le module de traitement est configuré pour recevoir depuis le module de réception la deuxième équation algébro-différentielle au cours d'une étape 304 et pour fusionner la seconde équation algébro-différentielle avec la première équation algébro-différentielle au cours d'une étape 308. Le module de traitement transmet ensuite le système d'équations algébro-différentielles au module de résolution au cours d'une étape 309 qui va résoudre ce système et obtenir un vecteur de variables d'état de sortie du procédé comprenant les deux opérations.

Selon un mode de réalisation où une opération est représentée à la fois par une équation algébriques explicite et une équation algébro-différentielle, le dispositif client sélectionne à l'étape 302 un mode de simulation. Si la simulation par l'intermédiaire de l'équation algébrique explicite est sélectionnée, le module de traitement effectue les étapes de création 305, d'injection 306 et fixation de la constante de temps 307 puis l'étape de fusion 308 avec les équations algébro-différentielles des autres opérations du procédé. Si la simulation par l'intermédiaire de l'équation algébro-différentielle est sélectionnée, le module de traitement fusionne directement cette équation algébro-différentielle avec les équations algébro-différentielles des autres opérations du procédé. Ainsi, la présente invention permet de combiner une simulation précise à l'aide d'une équation algébro-différentielle pour certaines opérations critiques et la sélection d'une simulation moins précise à l'aide d'une équation algébrique explicite. La solution de cette équation algébrique explicite étant intégrée dans une équation algébro-différentielle afin de permettre la fusion des équations algébro-différentielles dans un unique système de résolution.

## Le système de simulation

Un module de simulation tel que décrit ci-avant, et le procédé correspondant, peuvent être mis en oeuvre dans un système plus global et offrant un outil de conception industriel complet permettant à toute une équipe de conception de coopérer à l'évaluation et au développement d'un procédé chimique ou biochimique et l'installation industrielle correspondante.

Cet outil regroupe diverses fonctionnalités telle que : la gestion de données (par exemple des données expérimentales concernant des produits chimiques, des caractéristiques physico-chimiques relatives à des matières premières ou autre), le traitement de ces données, la définition d'unités opérationnelles pour réaliser un procédé chimique, la définition d'opérations de base entrant dans le procédé chimique, la définition d'une installation pour la mise en oeuvre du procédé avec des équipement matériels, l'évaluation économique et financière.

Là où toutes ces fonctionnalités étaient, dans l'art antérieur, gérées de manière indépendante et sans cohérence, elles sont regroupées dans un système selon l'invention et mises en commun afin d'achever le même but. Toutes ces fonctionnalités sont mises en oeuvre par des modules et regroupées dans un système tout en assurant une communication entre les modules.

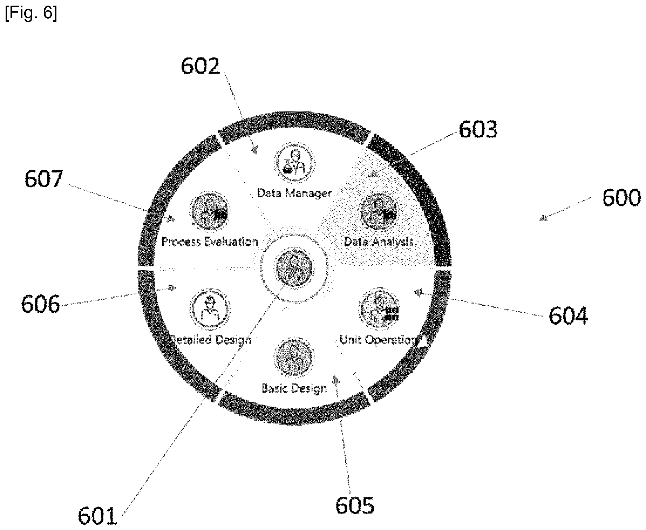

La figure 6 **illustre** schématiquement un système 600 selon des modes de réalisation. Il comporte un module central 601 capable de piloter le système et coordonner l'exécution des différents modules. Le système comporte en outre des modules 602 à 607 correspondant aux diverses fonctionnalités listées ci-dessus.

Par exemple, chacun des modules 604 à 606 relatifs à la définition d'unités opérationnelles, la définition d'opérations de base et de définition d'une installation chimique peuvent mettre en oeuvre un module de simulation 200 tel que décrit ci-avant. Le module client 201 tel que décrit ci-avant peut alors être le module central 601. Le module 602 permet de stocker et d'organiser des données expérimentales dans une structure de données pouvant être exploitée par tous les autres modules du système. En particulier, ce module offre des données relatives à des espèces chimiques dans lesquelles les matières premières, les réactifs et les produits vont être décomposés tout au long du processus. Le module 603 permet de traiter et d'analyser des données expérimentales, par exemple réaliser des analyses statistiques, identifier des paramètres d'un procédé chimique etc. Le module 607 permet de réaliser des estimations de performance en fonction des données expérimentales issues du module 602 ou du module 603 ou encore de résultats des simulations réalisées par les modules 604 à 606. Le module 607 permet aussi de réaliser des comparaisons entre différentes estimations de performance ou entre une estimation de performance et des données expérimentales.

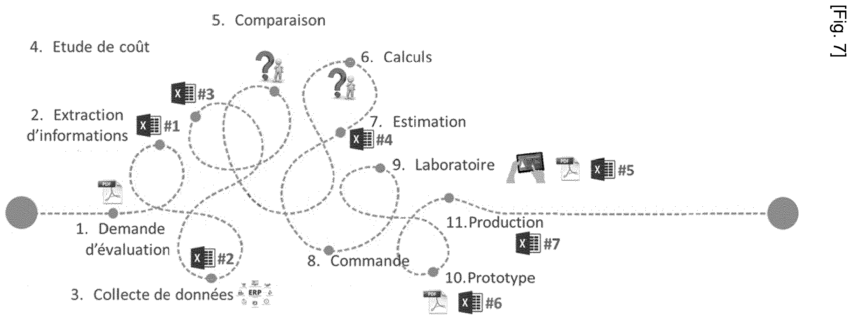

Une telle architecture permet de regrouper dans un même outil des informations et des fonctionnalités qui étaient éparpillés dans les modes de conception de l'art antérieur. En effet, selon l'art antérieur, comme illustré par la **figure 7,** beaucoup de temps et de ressources étaient gaspillées dans la collecte et la conversion de données éparses. Ainsi, lorsqu'une demande d'évaluation était reçue (1), il fallait tout d'abord en extraire les données techniques (2) (nature du procédé à définir, matières premières, produits, contraintes physico-chimiques, contraintes matérielles, contraintes de coûts etc.). Il fallait ensuite récupérer des données historiques et de savoir-faire permettant l'évaluation (3). Cette étape nécessitait déjà la communication entre différents acteurs et pouvait conduire à une perte d'information. Une fois les données internes et historiques récoltées, il fallait les traiter. Avant de lancer plus en avant l'évaluation, il fallait procéder à une étude de coûts (4), faisant intervenir de nouvelles équipes disposant de leurs propres outils et de leurs propres informations. C'est seulement après, qu'il était possible de commencer à réaliser des comparaisons (5) avec, éventuellement, des historiques de développement et des calculs (6) plus fins en termes de génie des procédés. Cette nouvelle étape faisait encore intervenir de nouvelles équipes avec leurs propres données et modes de calcul. Une fois les calculs obtenus, il était alors possible de procéder à une estimation complète (7) et de proposer un coût final de production. Une fois celle-ci communiquée à un client par exemple et une fois qu'une commande ferme était faite (8), les données utilisées étaient fournies au laboratoire chargé de procéder à la mise en oeuvre pratique du procédé défini et évalué (9). Un prototype était alors réalisé (10) pour un lancement en production à grande échelle (11).

Le problème majeur rencontré dans le processus de l'art antérieur décrit ci-avant est qu'à chaque étape les intervenant redéfinissaient les données et réalisaient leur traitement indépendamment de tout ce qui avait pu être fait avant. Cela conduisait à une perte d'efficacité et de précision.

Un système 600 selon les modes de réalisation de l'invention permet d'éviter ces difficultés en intégrant dans un même outil la gestion des données expérimentales, la prédiction par simulation mais aussi l'évaluation globale au moyen d'expériences passées. Cette approche innovante, désignée par GPX, acronyme de « Guess, Predict, Experimental » permet de réaliser des évaluations pertinentes et rapides.

Les modules 605 à 606 participent à l'approche « G » qui permet une évaluation globale sur la base d'une simulation à partir d'équations algébriques ou de données économiques passées. Les modules 602 à 607 participent à l'approche « P » en permettant une simulation précise, en utilisant des équations différentielles ou algébro-difftféren-tielles à partir de données expérimentales. Les modules 601, 602 et 607 participent à l'approche « X » en offrant des données expérimentales et des données historiques pour alimenter les autres modules des autres approches en données pertinentes.

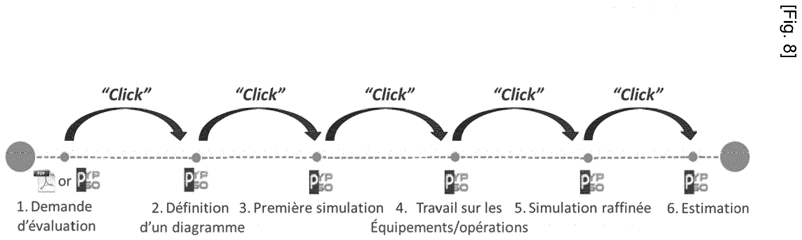

Comme illustré par la **figure 8,** une système selon des modes de réalisation permet ainsi, dans un même système de donnée et avec des traitements cohérents de réaliser toutes les opérations nécessaires à l'évaluation d'un procédé chimique ou d'une installation correspondant : réception ou définition d'une évaluation à réaliser avec les données techniques correspondantes (1), définition d'un diagramme de blocs avec des opérations et des équipements correspondant (2), une première simulation sur la base de ce diagramme (3), un raffinement des équipements et des opérations (4), une simulation plus précise (5) et une évaluation (6).

Un système selon des modes de réalisation permet ainsi de partager l'information sur un projet d'évaluation d'un procédé chimique, de capitaliser sur l'information collectée au fil des évaluations et de communiquer le résultat de ces évaluations. Tout cela est permis sans perte d'information et avec une grande cohérence dans les traitements.

D'une manière générale, les différents modules du système fonctionnent comme décrit ci-après.

Le module 602 permet d'organiser les données qui sont partagées dans le système. En particulier, il permet l'enregistrement de données scientifiques expérimentales concernant des espèces chimiques. Le format dans lequel ces données sont enregistrées est partagé entre tous les modules, ce qui permet un traitement efficace et coordonné entre les modules. En particulier, les modules mettant en oeuvre des simulations 604, 605 et 606 partagent ce format de données car ils accèdent aux données expérimentales, en particulier, comme cela est décrit ci-après, à la décomposition des matières premières utilisées par un procédé chimique. Cela est aussi le cas du module 607 qui permet les comparaisons, en particulier entre les résultats de simulation et des données expérimentales.

Lors du lancement d'une simulation, un projet est défini au sein du système au moyen d'un fichier « projet » et d'un ensemble de fichiers. un premier fichier décrit la structure du procédé chimique ou biochimique, un fichier décrit la séquence des opérations du procédé et le dernier représente la modélisation du procédé. Ensuite, différents fichiers sont créés pour représenter les simulations de chaque d'unité opérationnelle, opération de base et installation.

Une caractéristique particulière du système réside dans le fait que dans chaque fichier, il est possible de retrouver la décomposition des matières premières et des réactifs intermédiaires décomposés selon des espèces chimiques dont les caractéristiques se trouvent stockée via le module 602.

Ainsi, selon la fonction de chaque module, les accès en écriture et en lecture seront différents pour chaque type de fichier. Par exemple seul le module central 601 pourra accéder au ficher « projet en écriture ». Les autres modules ne pourront y accéder qu'en lecture pour les besoins de l'exécution de leurs fonctionnalités.

Le module 602 sera lui le seul à accéder aux fichiers représentatifs des données expérimentales en écriture. Les autres modules ne pourront y accéder qu'en lecture. Le module 603 quant à lui ne dispose pas de droits en écriture dans les fichiers descriptifs du projet. Il a par contre accès en lecture au fichier « projet » et aux fichiers représentant les données expérimentales. Les modules pouvant réaliser des simulations 604, 605, 606 ont quant à eux accès aux fichier projet et aux fichiers représentant les données expérimentales. Ils peuvent en outre accéder en écriture aux fichiers représentant les simulations.

Ainsi, pour chaque type de fichier seulement un unique module est en mesure d'y accéder en écriture. Par contre, différents modules peuvent partager la lecture d'un même type de fichier (par exemple tous les modules peuvent accéder aux fichiers représentatifs des données expérimentales. Le partage des informations est ainsi facilité dans le système.

Le **fichier projet** peut prendre la forme d'un fichier de type XML (extensible markup language) avec trois parties. Une première partie représente la liste des espèces chimiques mises en oeuvre dans le projet. Une autre partie représente la liste des matières premières avec une identification et une composition en référence avec la liste des espèces. Ainsi, chaque matière première de la deuxième partie est décomposée en espèces chimiques de la deuxième partie. Une troisième partie comporte les autres éléments intervenant dans les réactions comme par exemple les catalyseurs, résines absorbeurs filtres etc. avec un identifiant respectif et quelques paramètres physico-chimiques.

Les identifiants donnés aux espèces chimiques, matières premières et autres sont valides pour le fichier projet et donc pour toutes les fonctions réalisés par les différents modules. Ainsi, tous les modules peuvent avoir accès à la même décomposition et faire des calculs sur cette décomposition.

Le procédé chimique est représenté sous la forme d'un diagramme de blocs, chaque bloc représentant une opération ou une installation. Les blocs sont liés entre eux en fonction des entrées et des sorties comme illustré par la figure 1.

Dans le système, chaque bloc est représenté par un ensemble de trois fichiers.

Un premier fichier, du type XML comporte deux parties. Une première partie comporte une liste des flux avec une identification respective et des entrées et sortie de flux. Une deuxième partie comporte des blocs de procédé avec des identifications respectives, une position et des listes d'entrées et de sorties correspondant à celles de flux de la première partie.

Un deuxième fichier décrit les opérations associées au procédé. Ce deuxième fichier, de type XML comporte une première partie avec les flux d'entrée du procédé (listés par leur identification selon le premier fichier) avec l'identification des matières premières qu'ils portent ainsi que leur quantité. Il comporte une deuxième partie avec les listes des blocs de procédé et leurs températures de fonctionnement.

Le troisième fichier, de type XML aussi, comporte la modélisation du procédé avec deux parties. Une première partie avec les flux d'entrée et les propriétés physiques de chaque phase du flux et la répartition des espèces dans chaque phase. Une deuxième partie avec les blocs de procédé et la liste des phases pour chaque flux d'entrée avec les propriétés physiques et la répartition des espèces chimiques. Si une réaction chimique a lieu dans les blocs, elle est décrite dans cette partie du fichier.

Ce découpage en trois fichiers est identique pour les modélisations par les modules 604, 605, 606.

Chaque type de description de processus a un **fichier de données expérimentales** correspondant. Ce fichier comporte quatre parties. La première partie comporte une description similaire à celle que l'on trouve dans le premier fichier décrivant les blocs et décrit ci-dessus. La deuxième partie (qui est optionnelle) comporte des données de modélisation, similaires à celle du troisième fichier décrivant les blocs décrit ci-dessus. La troisième partie comporte une description identique à celle du deuxième fichier décrivant les blocs et décrit ci-dessus. La quatrième partie sert à stocker des résultats de mesure.

Les **fichiers de simulation** issus des modules 604, 605, 606 sont aussi du type XML et comportent quatre parties. La première partie est une copie du fichier « projet ». La deuxième partie est une copie du premier fichier décrivant le bloc simulé. La troisième partie est une copie du deuxième fichier décrivant le bloc simulé. La quatrième partie est une copie du troisième fichier décrivant le bloc simulé. Une partie additionnelle peut comporter une description des équilibres de masse et d'énergie exprimée en fonction des flux pour la masse et des blocs pour l'énergie. En ce qui concerne les fichiers issus des modules 604 et 605, ils peuvent aussi comporter balance des masses et des descriptions d'évolution de variables d'état telles que la température, les débits etc.

Les **évaluations** telles que traitées par le module 607 sont décrites dans le système par un fichier qui regroupe toutes les données nécessaires pour le calcul des coûts et d'autres critères d'évaluation de performance. D'une manière générale ce fichier peut comporter des identifiants de flux dans la réaction ou l'installation simulée qui sont cohérents avec ceux des fichiers de simulation. Il peut en outre comporter des identifiants d'espèces de référence qui sont cohérents avec les fichiers de simulation. Enfin le fichier peut comporter des coûts unitaires pour les matières premières utilisées par le procédé ou l'installation simulée. Ces coûts sont renseignés pour chaque matière première identifiée de manière cohérente avec le fichier de simulation.

D'une manière générale, lorsqu'une évaluation correspond à une matière première ou une espèce, elle est identifiée en fonction des identifiant de ces matière première ou espèce. Si elle correspond à un flux, elle sera identifiée en fonction de l'identifiant du flux.

Le système selon des modes de réalisation traite les fichiers comme une fonction de transfert qui prend en arguments et en sorties certains de fichiers décrits ci-avant. Il est possible de traiter tous ces fichiers de manière cohérente en utilisant comme donnée commune les espèces dans lesquelles sont décomposées les matières premières. En effet, les données expérimentales traitent en général des données relatives aux espèces. Il en va de même pour les simulations numériques. Enfin, s'agissant des données économiques et financières, elles traitent plutôt des matières premières, mais qui sont décomposées en espèces.

Par exemple, le module 602 peut prendre comme entrées le fichier projet ainsi que d'autres données utilisateur et produire à partir de cela le ficher de données expérimentales. L'utilisation du fichier projet garanti que toutes les données utilisateur soient exprimées en termes d'espèces par rapport aux matières premières. Par exemple encore, le module 605 prend comme entrées le fichier projet ainsi que des données utilisateur (il peut aussi utiliser le fichier de données expérimentales ou autre). Il rend comme sortie les fichiers de simulation. Ici aussi, la définition par le fichier projet en termes d'espèces chimiques permet de rendre des résultats cohérents avec cette représentation. Par exemple encore, le module 607 prend en entrée les fichiers de simulations crées à partir du fichier projet et rend en sortie des fichiers d'évaluation.

## EXEMPLES

Dans ce qui suit, on décrit des exemples d'utilisation d'un système selon l'invention.

Globalement, le système va être mis en oeuvre selon trois étapes principales.

La première étape 1. est une définition des matières premières et des espèces. Elle comporte trois sous-étapes 1.1, 1.2, 1.3. Elle peut être réalisée en utilisant les modules 601 et 602.

La première sous-étape 1.1 est une définition par l'utilisateur des matières premières entrant dans le procédé. La deuxième sous-étape 1.2 est une décomposition des matières premières en espèces chimiques (ex. : la matière première « alcool azéotropique » est décomposée en ses espèces chimiques la constituant, à savoir 96 % d'éthanol et 4 % d'eau). La troisième sous-étape 1.3 est une entrée par l'utilisateur des espèces chimiques ou des matières biologiques produites dans le procédé.

La deuxième étape est une définition des Schéma de blocs d'opération. Elle comporte cinq sous-étapes 2.1 à 2.5. Elle peut être réalisée par le module 605.

Dans la première sous-étape 2.1, un schéma de blocs d'opération constitué de blocs d'opération est crée par un utilisateur ou à partir d'une recette de laboratoire. On dispose d'une bibliothèque BBOP de blocs d'opération BOP, chaque bloc d'opération présentant un nombre d'entrées et de sorties, en espèces, matières premières, ou énergie et représentant une opération élémentaire, telle qu'un mélange, une réaction, une séparation, un chauffage. Ces blocs d'opération BOP représentent des transformations des flux ou des quantités indépendamment du volume ou du temps que cela nécessite. Ces blocs d'opération BOP ne sont pas associés à des équipements et ne contiennent pas de notion de productivité.

Dans la deuxième étape 2.2, on trouve une prise en compte par le système des relations entre les entrées et les sorties de chacun des blocs d'opération BOP sélectionnés. L'utilisateur peut fournir ces relations en extrayant un modèle d'une première bibliothèque de modèles d'opération MOP1 et en précisant éventuellement des paramètres de ce modèle, par exemple un ratio entre un débit d'entrée et de sortie du bloc. Ces relations peuvent concerner divers paramètres tels que des températures, des pressions, des quantités ou des flux de matière, des concentrations dans différentes phases. Il s'agit à ce stade d'un mode dit « Guess » en terminologie anglo-saxonne : seuls l'intuition, l'expérience de l'utilisateur sont utilisés, aucune information physico-chimique n'est nécessaire.

Dans l'étape 2.3, au moyen du schéma de blocs d'opération et des relations entre les entrées et les sorties de chacun des blocs d'opération BOP sélectionnés, le système détermine des bilans globaux du procédé. Ces bilans globaux peuvent concerner la production des espèces chimiques ou des matières biologiques produites dans le procédé, la consommation de matières premières ou d'énergie. Cette détermination peut s'opérer indifféremment en continu (données de flux) ou en discontinu (données de quantité par lot). A ce stade, le système peut également déterminer une première estimation de critères de performance du procédé basée sur les consommations de matière premières et/ou d'énergie, ce critère pouvant être économique ou environnemental.

Dans l'étape 2.4, la possibilité est alors offerte à l'utilisateur de répéter l'étape 2.1, en sélectionnant d'autres blocs d'opération ou en les agençant autrement pour obtenir un autre schéma de blocs d'opération et/ou de répéter l'étape 2.2 en choisissant d'autres relations entre les entrées et les sorties des blocs d'opération sélectionnés, puis d'effectuer à nouveau l'étape 2.3 de détermination des bilans globaux, et de poursuivre jusqu'à obtention de bilans globaux satisfaisants.

Optionnellement, dans l'étape 2.5, la possibilité est offerte à l'utilisateur à l'issue d'une étape 2.3 ou 2.4 de remplacer les relations entre les entrées et les sorties de certains des blocs d'opération BOP par des relations extraites d'une seconde bibliothèque de modèles d'opération MOP2. Cette seconde bibliothèque de modèles d'opération MOP2 comporte des caractéristiques plus élaborées, tenant compte par exemple d'informations thermodynamiques, ou de composition de phases. Le système détermine alors les bilans globaux, suivant l'étape 2.3, sur base des caractéristiques améliorées. L'utilisateur sélectionnera avantageusement les blocs d'opération pour lesquels il faut remplacer les relations entrées/sorties parmi les blocs d'opération les plus critiques dans le procédé. A ce stade, le système peut également proposer une deuxième détermination des critères de performance basée sur les consommations de matière premières et/ou d'énergie.

La troisième étape est la définition de schémas d'équipements. Elle comporte six sous-étapes 3.1 à 3.6. Elle peut être réalisée par les modules 604, 606.

Dans la première sous-étape 3.1, on trouve une transformation du schéma de blocs d'opération en un schéma d'équipements représentant l'installation industrielle à concevoir. Des équipements sont choisis dans une bibliothèque d'équipements génériques BEQGEN ou dans une bibliothèque d'équipements spécifiques BEQSPEC pour réaliser les opérations d'un ou plusieurs blocs d'opération BOP du schéma de blocs d'opération, jusqu'à la transformation de tous les blocs d'opération du schéma de blocs d'opération. Les équipements de la bibliothèque BEQGEN sont des équipements génériques : leurs caractéristiques peuvent rester assez floues, voire idéalisées : un réacteur peut par exemple être défini comme un système adiabatique parfaitement mélangé, indépendamment des moyens pour parvenir à ce résultat. Les équipements de la bibliothèque BEQSPEC sont des équipements spécifiques. Ils peuvent alors avoir des caractéristiques bien précises et peuvent être associés à une référence constructeur donnée. La démarche présentée autorise également la création directe d'un schéma d'équipements sans création préalable d'un diagramme d'opérations. Un tel raccourci peut par exemple trouver son utilité si l'on cherche à représenter une installation déjà existante.

Dans une étape 3.2, on trouve une spécification des différentes opérations réalisées dans chaque équipement. Pour chaque opération, on spécifie en particulier sa nature (chargement de matière première, réaction, vidange, changement de température, etc.) ainsi que sa durée. L'ensemble de ces spécifications d'équipements correspond à une procédure opératoire telle que celles utilisées dans les ateliers industriels. Cette procédure opératoire n'implique pas d'éléments de modélisation.

L'étape 3.3 est une détermination par le système de paramètres de fonctionnement de l'installation industrielle représentée par le schéma d'équipement à l'aide des bibliothèques de modèles MOP1 et MEQ1 ainsi que de la connectivité entre les équipements. Les modèles de la bibliothèque MEQ1 servent à décrire les opérations effectuées dans les équipements telles que saisies à l'étape 3.2. De même que pour la bibliothèque MOP1, ce sont des modèles basés sur une description macroscopique des phénomènes et ne faisant appel à aucune donnée physico-chimique. Les paramètres de fonctionnement de l'installation industrielle peuvent comprendre des productivités, des rendements, des paramètres de qualités des produits, tels que la pureté, la production de déchets, la consommation d'énergie. A ce stade, le système peut déterminer une première évaluation d'un critère de performance basé à la fois sur la consommation de matières premières, d'énergie, la taille et le mode d'opération des équipements. Ce critère peut par exemple être économique ou environnemental.

A l'étape 3.4, la possibilité est alors offerte à l'utilisateur de répéter l'étape 3.1, en sélectionnant d'autres équipements, et/ou de répéter l'étape 3.2 en choisissant d'autres caractéristiques pour les opérations réalisées dans les équipements, puis d'effectuer à nouveau l'étape 3.3 de détermination de paramètres de fonctionnement, et de poursuivre jusqu'à obtention de paramètres respectant les contraintes fixées.

Optionnellement, à l'étape 3.5, la possibilité est offerte à l'utilisateur à l'issue d'une étape 3.3 ou 3.4 de remplacer les caractéristiques de certaines opérations effectuées dans des équipements génériques ou spécifiques du schéma d'équipement par des caractéristiques extraites d'une seconde bibliothèque MEQ2 de modèles d'opérations effectuées dans des équipements. Les modèles issus de cette seconde bibliothèque sont nettement plus élaborés et permettent par exemple de calculer des conversions dans des réacteurs ou des performances de séparation en distillation à partir de données physico-chimiques. Le système détermine alors à nouveau des paramètres de fonctionnement de l'installation industrielle, suivant l'étape 3.3, sur base des caractéristiques améliorées. A ce stade, le système peut déterminer des critères de performance basée à la fois sur la consommation de matières premières, d'énergie, la taille et le mode d'opération des équipements.

Optionnellement, à l'étape 3.6, la possibilité est offerte à l'utilisateur à l'issue d'une étape 3.3 ou 3.4 de remplacer les caractéristiques de certaines opérations effectuées dans des équipements génériques ou spécifiques du schéma d'équipement par des caractéristiques extraites d'une troisième bibliothèque MEQ3 de modèles d'opérations effectuées dans des équipements. Les modèles issus de cette troisième bibliothèque sont des modèles détaillés et permettent par exemple de représenter des non-idéalités de mélange ou des phénomènes hydrodynamiques complexes via la prise en compte de telle ou telle caractéristique géométrique d'équipements spécifiés. Le système détermine alors à nouveau des paramètres de fonctionnement de l'installation industrielle, suivant l'étape 3.3, sur base des caractéristiques améliorées. A ce stade, le système peut déterminer des critères de performance basée à la fois sur la consommation de matières premières, d'énergie, la taille et le mode d'opération des équipements. Cette sous-étape peut être réalisée par le module 607.

**Exemple 1**

On décrit maintenant deux exemples d'utilisation pour simuler un projet.

Un **premier exemple** d'application de ce procédé de simulation est donné ci-après. Il est inspiré d'un cas réel et concerne une purification située en aval d'une synthèse organique qui produit de la delta-valerolactone. L'objectif est de minimiser le coût de production de la delta-valerotlactone. Cet objectif peut se décliner dans les deux questions suivantes : peut-on proposer des améliorations simples à la technologie de purification actuelle ? Peut-on proposer d'autres technologies de purification avec des coûts inférieurs ?

Comme exposé dans la section ci-dessous, l'information de départ est restreinte, ce qui empêche tout recours à des modèles élaborés (que ce soit en matière de thermodynamique ou de cinétique) à moins de recourir à des campagnes de mesures longues et coûteuses. L'approche descendante ici exposée consiste à réaliser une première série de calculs sur la base des informations disponibles ou accessibles immédiatement et gratuitement. Puis, sur la base de ces calculs, on identifie les informations supplémentaires réellement nécessaires afin de minimiser l'effort de collecte de cette information.

En fin de réaction, la delta-valerolactone, se trouve dans une solution aqueuse qui contient également de la cyclopentanone, du peroxyde d'hydrogène et de l'acide acétique. On cherche à retirer ces deux dernières espèces et à déshydrater le produit. La procédure traditionnelle consiste à : neutraliser le peroxyde avec du sulfite de sodium, neutraliser l'acide acétique avec du carbonate de sodium, extraire les molécules organiques à l'aide d'un solvant organique, évaporer l'eau pour faire précipiter les sels, filtrer les sels, évaporer le solvant organique. Nous envisagerons également une configuration basée sur la neutralisation thermique du peroxyde et le retrait de l'acide acétique par distillation.

La première étape est la définition des matières premières et des espèces.

Les espèces chimiques mises en jeu dans les procédés étudiés sont listées dans la Table 2. Certaines d'entre elles proviennent des matières premières listées dans la Table 1, d'autres sont générées par réaction chimique.

Pour chacune des espèces de la Table 2, sont fournies quelques informations que n'importe qui peut trouver gratuitement et rapidement.

**Table 1 : matières premières et espèces injectées dans les procédés de purification de la DVL**

| Matières premières | Espèces associées (% masse) |
| --- | --- |
| Mélange de réaction | 54,01 % $H_2O$ ; 1,11% CP ; 3,48% $H_2O_2$ ; 16,27% AcH ; 25,13% DVL |
| MIBK fraiche | 100% MIBK |
| Sulfite de sodium 20% | 80% $H_2O$ ; 20% $Na_2SO_3$ |
| Carbonate de sodium 20% | 80% $H_2O$ ; 20% $Na_2CO_3$ |
| **Soit 4 matières premières** | **Soit 8 espèces associées** |

**Table 2 : liste des espèces mises en jeu dans les procédés de purification de la DVL**

| Nom courant ou formule | CAS | Nom dans ce document | Masse molaire (g/mol) | Point d'ébullition normal (°C) |
| --- | --- | --- | --- | --- |
| cyclopentanone | 120-92-3 | CP | 84.12 | 131 |
| delta-valerolactone | 542-28-9 | DVL | 100.12 | 230 |
| Eau | 7732-18-5 | H2O | 18.02 | 100 |
| $H_2O_2$ | 7722-84-1 | H2O2 | 34.01 | - |
| Acide acétique | 64-19-7 | AcH | 60.05 | 118 |
| Sulfite de Sodium | 7757-83-7 | Na2SO3 | 126.04 | - |
| Carbonate de Sodium | 497-19-8 | Na2CO3 | 105.99 | - |
| $CO_2$ | 124-38-9 | CO2 | 44.01 | - |
| Acétate de Sodium | 127-09-3 | AcNa | 82.03 | - |
| Sulfate de Sodium | 7757-82-6 | Na2SO4 | 142.04 | - |

(suite)

| Nom courant ou formule | CAS | Nom dans ce document | Masse molaire (g/mol) | Point d'ébullition normal (°C) |
|---|---|---|---|---|
| MIBK | 108-10-1 | MIBK | 100.16 | 116 |
| Dioxygène | 7782-44-7 | O2 | 32.00 | - |

La deuxième étape est la définition de schémas de blocs d'opération.

Dans l'approche décrite ci-dessus, l'étape 2.1 pour la modélisation d'un processus est l'élaboration d'un schéma de blocs d'opération. Chaque icône de ce schéma représente une opération (c'est-à-dire une action sur un flux ou sur une quantité). A l'étape 2.2, ces opérations sont décrites par les relations entre leurs entrées et leurs sorties et peuvent être représentées par des modèles empiriques très simples ou par des modèles thermodynamiques plus précis. A l'étape 2.3, la résolution des équations de bilans associées aux relations entre les entrées et les sorties de chaque bloc et à la connectivité des blocs entre eux nous permettra de connaitre les flux (ou quantités) de masse et de chaleur à chaque endroit du système, et en particulier en sortie.

Les trois schémas d'opération considérés sont représentés :

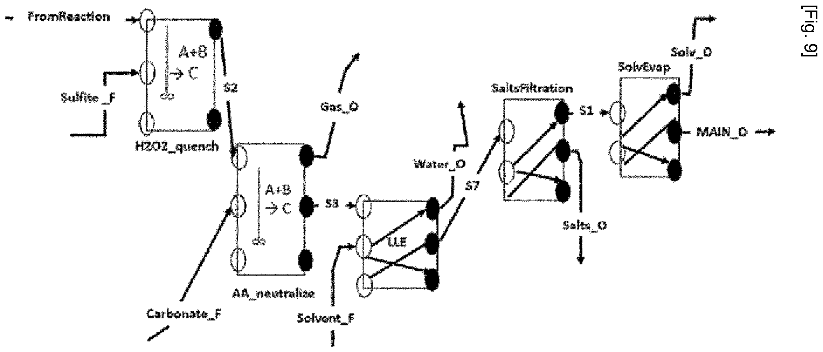

- Sur la figure 9 : schéma classique de purification par neutralisation saline et extraction liquide-liquide sans recyclage de solvant (cas 1)

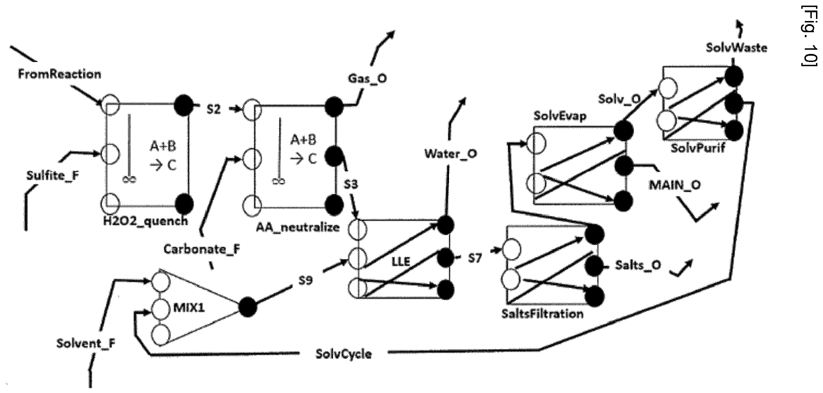

- Sur la figure 10 : schéma alternatif de purification par neutralisation saline et extraction liquide-liquide avec recyclage de solvant (cas 2)

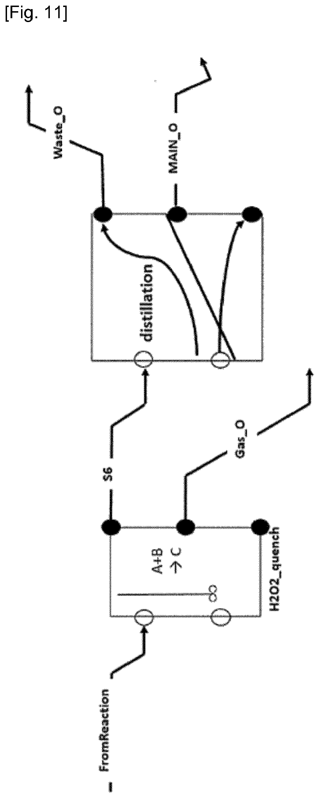

- Sur la figure 11 : schéma alternatif de purification par neutralisation thermique et distillation (cas 3)

Les schémas de blocs d'opération peuvent aussi bien se lire en flux (kg/h) pour les procédés continus qu'en quantité (kg) traitée par opération pour les procédés discontinus.

Dans l'étape 2.2, des relations entre les entrées et les sorties des blocs d'opérations sont définies.

Les modèles de la bibliothèque MOP1 proposent une description très macroscopique de l'effet de chaque bloc d'opération sur les flux ou quantités traversantes. Ce descriptif macroscopique se prête bien à l'utilisation d'hypothèses basées sur l'intuition et l'expérience.

Le schéma de la figure 9 comporte deux réactions *H2O2_quench* et *AA_neutralize* correspondant respectivement à la neutralisation de H2O2 par Na2SO3 et de AcH par Na2CO3. Ce schéma comporte également trois séparations *LLE, SaltsFiltration* et *SolvEvap* correspondant respectivement à l'extraction de la DVL et de la CP par un solvant organique (avec retrait de l'eau et précipitation des sels), la filtration des sels, l'évaporation du solvant.

Le schéma de la figure 10 est extrêmement similaire à celui de la figure 1 avec en plus l'opération *SolvPurif* - destinée à séparer le solvant à jeter de celui pouvant être recyclé - ainsi que l'opération *MIX1* destinée à mélanger le solvant frais et le solvant recyclé.

Le schéma de la figure 11 comporte simplement le bloc réaction *H2O2_quench* correspondant à la neutralisation thermique de H2O2 et le bloc séparation *distillation* correspondant au retrait de H2O et AcH.

De façon générale, les opérations de mélange consistent simplement à regrouper les masses (ou débits massiques) de chaque espèce contenue dans l'ensemble des flux entrants. Pour les opérations de séparations et de réaction possédant plusieurs flux de sortie, un ratio est fourni par l'utilisateur pour chaque espèce afin d'indiquer sa répartition entre les flux de sortie. Dans les exemples ci-après ces ratios sont souvent de 0% ou 100% mais ils peuvent prendre n'importe quelle valeur entre 0% et 100%. Par ailleurs, ce système de ratios est ici illustré sur des situations à deux flux sortants mais il s'applique également sur des situations où ces flux seraient plus nombreux ainsi que sur des équilibres entre phases.

Les phénomènes réactifs représentés par les blocs de réactions sont décrits dans la Table 3.

**Table 3 : modèles de réaction**

| Nom | Equation | Conversion |
|---|---|---|
| H2O2_quench (cas 1 et 2) | $H_2O_2 + Na_2SO_3 \rightarrow H_2O + Na_2SO_4$ | 100% de H2O2 |
| AA_neutralize | $2\,AcH + Na_2CO_3 \rightarrow 2\,AcNa + H_2O + CO_2$ | 100% de AcH |
| H2O2_quench (cas 3) | $2\,H_2O_2 \rightarrow 2\,H_2O + O_2$ | 100% de H2O2 |

La table ci-dessous comporte des ratios de partitions correspondant à l'hypothèse selon laquelle, lors de la neutralisation chimique de AcH, tout le $CO_2$ généré est évacué sans entrainement de liquide. Dans la **Table 9,** une hypothèse identique est réalisée pour le dégagement de 02 dans le cas d'une neutralisation thermique.

**Table 4 : ratios de partition des espèces entre les sorties de *AA_neutralize***

|  | Gas_O | S3 |
|---|---|---|
| CO2 | 100% | 0% |
| Autres espèces | 0% | 100% |

Les ratios du tableau ci-dessous représentent une extraction liquide-liquide où les phases aqueuses et organiques sont totalement immiscibles. Pour le retrait de l'eau, toute la charge de l'opération est portée à 100°C, l'eau est ainsi évaporée (enthalpie de changement d'état de 2260 kJ/kg). Puis tous les flux sortants sont ramenés à 25°C à l'état liquide.

**Table 5 : ratios de partition des espèces entre les sorties de *LLE***

|  | Water_O | S7 |
|---|---|---|
| H2O, CO2 | 100% | 0% |
| Autres espèces | 0% | 100% |

Pour le retrait des sels (cas 1 et 2), on suppose une filtration sans rétention de liquide dans le solide retenu.

**Table 6 : ratios de partition des espèces entre les sorties de *SaltsFiltration***

|  | Salts_O | S1 |
|---|---|---|
| Na2SO3, Na2CO3, AcNa, Na2SO4 (solides) | 100% | 0% |
| Autres espèces | 0% | 100% |

Pour l'opération SolvEvap, on considère que toute la charge est portée à 120°C et que la MIBK est ainsi évaporée puis que les deux flux sortants sont ramenés à 25°C sous état liquide. L'enthalpie de changement d'état est prise égale à 400 kJ/kg.

**Table 7 : ratios de partition des espèces entre les sorties de *SolvEvap***

|  | Solv_O | MAIN_O |
|---|---|---|
| MIBK | 100% | 0% |
| CP, DVL | 0% | 100% |
| Autres espèces | 50% | 50% |

Dans le procédé de la figure 10, on considère qu'une partie du solvant ne peut pas être réutilisée et que le recyclage concerne 95% du solvant (voir **Table 8).** De même que pour SolvEvap, on considère une évaporation à 120°C du flux de MIBK.

**Table 8 : ratios de partition des espèces entre les sorties de *SolvPurif***

|  | SolvWaste | SolvCycle |
|---|---|---|
| MIBK | 5% | 95% |
| Autres espèces | 100% | 0% |

**Table 9 : ratios de partition des espèces entre les sorties de *H2O2_quench* dans le cas de la neutralisation thermique**

|  | Gas_O | S6 |
|---|---|---|
| O2 | 100% | 0% |
| Autres espèces | 0% | 100% |

**Table 10 : ratios de partition des espèces entre les sorties de *distillation***

|  | Waste_O | MAIN_O |
|---|---|---|
| O2, H2O, AcH | 100% | 0% |
| CP, CVL, AcNa | 0% | 100% |
| H2O2 | 0% | 100% |

Le système de ratios de partition est dans le cas présent utilisé pour rendre compte de séparation généralement idéales. De par sa structure, ce système permet de garder conscience de cette hypothèse d'idéalité et autoriserait à représenter des écarts à l'idéalité, par exemple en prenant des ratios 97%/3% au lieu des 100%/0% actuels.

Les bilans globaux sont ensuite déterminés à l'étape 2.3.

Dans les trois configurations, on cherche à traiter une charge de 4050 kg de mélange réactionnel (voir composition dans la Table 1).

Les débits de sulfite et de carbonate sont calculés de façon à neutraliser totalement H2O2 et AcH. Le débit de MIBK fraiche est calculé de façon à ce que dans *LLE,* on mélange 4000 kg de MIBK avec la charge de DVL et de CP.

Dans le cas de base, on obtient le bilan de matière de la Table 11 et le bilan d'énergie de la Table 12.

**Table 11 : description des flux du procédé dans le cas de base (cas 1)**

|  | Flux | Matières premières | Quantités |
|---|---|---|---|
| SORTANTS | *MAIN_O* |  | 1063 kg |
|  | *Gas_O* |  | 242 kg |
|  | *Water_O* |  | 6921 kg |
|  | *Salts_O* |  | 1525 kg |
|  | *Solv_O* |  | 4000 kg |
| INTRANTS | *FromReaction* | Mélange de réaction | 4050 kg |
|  | *Sulfite_F* | Sulfite de sodium 20% | 2700 kg |
|  | *Carbonate_F* | Carbonate de sodium 20% | 3000 kg |
|  | *Solvent_F* | MIBK fraiche | 4000 kg |
| INTERNES | *S2* |  | 6750 kg |
|  | *S3* |  | 9509 kg |
|  | *S7* |  | 6599 kg |
|  | *S1* |  | 5063 kg |

**Table 12 : échanges thermiques dans le cas de base (cas 1**

| Bloc opération | Sens de l'échange | Energie (kWh) |
|---|---|---|
| *H2O2_quench* | Apport au procédé | 259 |
| *AA_neutralize* | Apport au procédé | 68 |

(suite)

| Bloc opération | Sens de l'échange | Energie (kWh) |
|---|---|---|
| *LLE* | Apport au procédé | 4808 |
| | Retrait du procédé | 5197 |
| *SolvEvap* | Apport au procédé | 678 |
| | Retrait du procédé | 678 |

Dans le cas avec recyclage du solvant d'extraction, on obtient les résultats de la **Table 13** et de la **Table 14.** Comparativement au cas de base, les principales différences interviennent au niveau des entrées et sorties de solvant. Le recyclage permet de les réduire considérablement moyennant un apport d'énergie supplémentaire.

**Table 13 : description des flux du procédé dans le cas avec recyclage du solvant d'extraction (cas 2)**

| | Flux | Matières premières | Quantités |
|---|---|---|---|
| SORTANTS | *MAIN_O* | | 1063 kg |
| | *Gas_O* | | 242 kg |
| | *Water_O* | | 6921 kg |
| | *Salts_O* | | 1525 kg |
| | *SolvWaste* | | 200 kg |
| INTRANTS | *FromReaction* | Mélange de réaction | 4050 kg |
| | *Sulfite_F* | Sulfite de sodium 20% | 2700 kg |
| | *Carbonate_F* | Carbonate de sodium 20% | 3000 kg |
| | *Solvent_F* | MIBK fraiche | 200 kg |
| INTERNES | *S2* | | 6750 kg |
| | *S3* | | 9509 kg |
| | *S7* | | 6599 kg |
| | *S1* | | 5063 kg |
| | *Solv_O* | | 4000 kg |
| | *SolvCycle* | | 3800 kg |
| | *S9* | | 4000 kg |

**Table 14 : échanges thermiques dans le cas du procédé avec recyclage du solvant d'extraction (cas 2)**

| loc opération | Sens de l'échange | Energie (kWh) |
|---|---|---|
| *H2O2_quench* | Apport au procédé | 259 |
| *AA_neutralize* | Apport au procédé | 68 |
| *LLE* | Apport au procédé | 4808 |
| | Retrait du procédé | 5197 |
| *SolvEvap* | Apport au procédé | 678 |
| | Retrait du procédé | 678 |
| SolvPurif | Apport au procédé | 600 |
| | Retrait du procédé | 600 |

Dans le cas du procédé avec neutralisation thermique du peroxyde, on obtient le bilan matière de la **Table 15** et le bilan d'énergie de la **Table 16.** Les quantités de matière et d'énergie sont sensiblement inférieures à celles des autres configurations. En effet, on n'apporte ni solvant d'extraction, ni eau (avec le sulfite et le carbonate).

**Table 15 : description des flux du procédé dans le cas de la neutralisation thermique du peroxyde (cas 3)**

|  | Flux | Matières premières | Quantités |
|---|---|---|---|
| SORTANTS | MAIN_O | | 1063 kg |
| | Gas_O | | 66 kg |
| | Waste_O | | 2921kg |
| INTRANTS | FromReaction | Mélange de réaction | 4050kg |
| INTERNES | S6 | | 3984 kg |

**Table 16 : échanges thermiques dans le cas de la neutralisation thermique du peroxyde (cas 3)**

| Bloc opération | Sens de l'échange | Energie (kWh) |
|---|---|---|
| H2O2_quench | Apport au procédé | 315 |
| distillation | Apport au procédé | 1883 |
| | Retrait du procédé | 2157 |

Ces six tableaux constituent trois résultats de simulation.

Pour l'étape 2.4, lorsque l'on dispose d'une recette de laboratoire - c'est-à-dire d'une procédure expérimentale éventuellement accompagnée d'éléments de résultats - le système sur lequel porte cette invention permet la saisie des informations présentes dans la recette. Il est possible de saisir toutes les informations de la recette sans préjuger de leur éventuelle utilisation ultérieure ; en parallèle aucune information ne doit impérativement être fournie hormis la nature de chaque opération. Une fois cette recette saisie, le programme d'ordinateur est capable d'en interpréter le contenu pour créer la structure d'un diagramme de blocs-opérations sur la base de ce contenu.

La recette de laboratoire suivante peut être utilisée comme base pour créer le diagramme d'opérations de la figure 9.

- Ajouter 4,05 kg de *mélange de réaction* et 2,75 kg de *sulfite de sodium 20%.* Laisser réagir durant 2 heures
- Ajouter 3,5 kg de *carbonate de sodium 20%,* laisser réagir durant 1 heure. Un dégagement gazeux est observé
- Ajouter 4 kg de *MIBK fraiche.* Mélanger et laisser décanter. Evaporer la phase aqueuse à 100°C. Une précipitation est observée.
- Filtrer le mélange
- Concentrer par évaporation du solvant à 120°C

Une recette de laboratoire peut par exemple provenir d'un article scientifique relatif à une expérience de synthèse. Cet article constituant alors l'un des premières sources d'information de la démarche.

Pour l'évaluation des coûts de l'étape 2.5, les bilans des six tableaux précédents vont à présent servir de base à des calculs économiques, ils permettent de calculer la part variable du coût de production. Ces coûts seront rapportés au kilogramme de DVL dans le flux *MAIN_O*.

Les hypothèses utilisées sont exposées ci-dessous. Pour beaucoup, ce sont des ordres de grandeur issus de l'expérience.

**Table 17 : coût unitaire des matières premières**

| Matière première | Prix (€/kg) |
|---|---|
| Mélange de réaction | 7.00 |
| Sulfite de sodium 20% | 0.38 |
| Carbonate de sodium 20% | 0.25 |
| MIBK fraiche | 0.98 |

**Table 18 : coût de traitement des sorties secondaires de matière**

| Flux | Coût de traitement (€/kg) |
|---|---|
| Gas_O (tous les cas) | 0.00 |
| Water_O (cas 1 et 2) | 0.15 |
| Salts_O (cas 1 et 2) | 0.10 |
| Solv_O (cas 1) | 0.15 |
| SolvWaste (cas 2) | 0.15 |
| Waste_O (cas 3) | 0.15 |

Les flux d'énergie apportés aux procédés (chauffage/évaporation) se voient affecter un coût de 0.10 €/kWh. Les flux d'énergie retirés des procédés (refroidissement/condensation) se voient affecter un coût de 0.05 €/kWh. Les bilans de matière portent sur des espèces, l'économie sur la valeur des matières premières d'où l'importance de l'étape 1.2.

Sur la base des bilans de matière et d'énergie ainsi que des hypothèses économiques ci-dessus, on aboutit aux coûts variables des trois versions du procédé tels qu'exposés dans la Table 19.

**Table 19 : contribution de chaque entrée à la partie variable du coût de production de DVL. Les valeurs sont exprimées en €/kg par kg de DVL dans le flux *MAIN_O***

| | Procédé 1 | Procédé 2 | Procédé 3 |
|---|---|---|---|
| | Cas de base sans recyclage | Recyclage du solvant d'extraction | Neutralisation thermique |
| Mélange de réaction | 27.86 | 27.86 | 27.86 |
| Sulfite de sodium 20% | 1.01 | 1.01 | - |
| Carbonate de sodium 20% | 0.74 | 0.74 | - |
| MIBK fraiche | 3.85 | 0.19 | - |
| Traitement d'effluents | 1.76 | 1.20 | 0.43 |
| Energie | 0.86 | 0.95 | 0.32 |
| TOTAL | 36.07 | 31.94 | 28.61 |
| Ecart au cas de base | - | -11,4% | -20,7% |

On remarque que le procédé 2 permet, grâce au recyclage de solvant, des économies intéressantes à la fois au niveau de la MIBK fraiche et du traitement d'effluents. La contrepartie au niveau de l'énergie est minime.

Le procédé 3 permet des économies encore plus importantes car la technique de neutralisation employée réduit nettement les quantités de matières premières à injecter et les quantités de solvants (eau y compris) à séparer et retraiter.

Si l'on compare les procédés 1 et 2, on constate un net écart de coûts variables. Or, sachant que les deux configurations sont extrêmement proches, on peut supposer que le dispositif permettant le recyclage n'induira pas un supplément de coûts fixes suffisamment significatif pour compenser l'avantage du procédé 2 en matière de coûts variables. On peut donc considérer que le procédé 1 sera nécessairement moins compétitif que le procédé 2, il ne sera donc plus considéré pour la suite de l'étude.

Si l'on compare les procédés 2 et 3, on constate que le procédé 3 est nettement plus avantageux en matière de coûts variables. Toutefois, au vu des différences fondamentales (nature des réactions chimiques, structure du procédé, etc.) entre ces deux procédés, il apparaît nécessaire, pour tirer une conclusion sérieuse, de prendre en compte les coûts fixes et, à travers eux, les notions de temps et d'équipement.

Dans l'étape 3.1, chacun des blocs d'opération de deux schémas conservés est affecté à un équipement. Notons que plusieurs opérations peuvent être réalisés dans un même équipement (par exemple les deux réactions de neutralisation dans le cas du procédé 2).

Le système dispose d'une bibliothèque d'équipements génériques BEQGEN et d'une bibliothèque d'équipement spécifiques BEQSPEC, chaque équipement ayant ses caractéristiques.

Dans le cas présent (voir Table 20 et Table 21), tous les équipements concernés proviennent de la bibliothèque BEQGEN. Dans le cas des réacteurs, un premier choix technologique est effectué puisque l'on décide d'employer des réacteurs agités, choix très classique dans le cas de procédés discontinus. Dans le cas des séparateurs, on utilise pour l'instant des séparateurs génériques. Ainsi, il n'est pas nécessaire de prendre d'emblée position sur des questions telles que la nature du filtre ou le nombre de plateaux de la colonne de distillation. Nous sommes toutefois conscients que le choix effectué limite pour l'instant la précision des résultats relatifs aux équipements de séparation.

Une première valeur de volume (un peu arbitraire) est donnée pour chaque équipement. Celle-ci pourra être modifiée par la suite.

**Table 20 : affectation des opérations en équipements dans le cas du procédé 2 (recyclage du solvant d'extraction)**

| Bloc d'opération | Equipement | | |
|---|---|---|---|
| | Nom | Type | Volume (m$^3$) |
| H2O2_quench | QuenchSTR | Réacteur agité | 14 |
| AA_neutralize | | | |
| LLE | LL_extractor | Séparateur générique | 18 |
| SaltsFiltration | SaltsFilter | Séparateur générique | 10 |
| SolvEvap | Evaporator | Séparateur générique | 8 |
| SolvPurif | SolvPurif | Séparateur générique | 6 |
| MIX1 | SolventTank | Cuve | 6 |

**Table 21 : affectation des opérations en équipements dans le cas du procédé 3 (neutralisation thermique)**

| Bloc d'opération | Equipement | | |
|---|---|---|---|
| | Nom | Type | Volume (m$^3$) |
| H2O2_quench | QuenchSTR | Réacteur agité | 6 |
| distillation | Distill | Séparateur générique | 6 |

Pour l'étape 3.2, on remarque qu'au démarrage du procédé 2 (extraction liquide-liquide avec recyclage de solvant), l'équipement *SolventTank* contient 3800 kg de MIBK. Ensuite la procédure opératoire appliquée est celle exposée dans la Table 22. Pour un certain nombre d'opérations, une durée arbitraire de 0,01h est appliquée car on présume que la durée de ces étapes est négligeable au regard des durées de réactions et de séparations.

**Table 22 : procédure opératoire du procédé 2 (extraction liquide-liquide avec recyclage du solvant)**

| Ordre | Opération | Durée (h) |
|---|---|---|
| 1 | Chargement de 4050 kg de *Mélange réactionnel* dans *QuenchSTR* | 0,01h |
| 2 | Chargement de 2750 kg de *Sulfite de sodium 20%* dans *QuenchSTR* | 0,01h |
| 3 | Réaction dans *QuenchSTR* | 2h |
| 4 | Chargement de 3500 kg de *Carbonate de sodium 20%* dans *QuenchSTR* | 0,01h |
| 5 | Réaction dans *QuenchSTR* | 1h |
| 6 | Vidange partielle de *QuenchSTR* par *Gas_O* | 0,01h |
| 7 | Vidange totale de *QuenchSTR* dans *LL_extractor* | 0,01h |
| 8 | Chargement de 200 kg de *MIBK fraiche* dans *SolventTank* | 0,01h |

(suite)

| Ordre | Opération | Durée (h) |
|---|---|---|
| 9 | Vidange totale de *SolventTank* dans *LL_extractor* | 0,01h |
| 10 | Agitation dans *LL_extractor* | 4h |
| 11 | Vidange partielle de *LL_extractor* par *Water_O* | 1h |
| 12 | Vidange totale de *extractor* dans *SaltsFilter* | 0,01h |
| 13 | Vidange partielle de *SaltsFilter* dans *Evaporator* | 2h |
| 14 | Vidange totale de *SaltsFilter* par *Saits_O* | 0,01h |
| 15 | Vidange partielle de *Evaporator* dans *SolvPurif* | 2h |
| 16 | Vidange partielle de *SolvPurif* dans *SolventTank* | 2h |
| 17 | Vidange totale de *Evaporator* par *MAIN_O* | 0,01h |
| 18 | Vidange totale de *SolvPurif* par *SolvWaste* | 0,01h |

Pour le procédé 3 (neutralisation thermique), la procédure opératoire est exposée dans la Table 30.

**Table 23 : procédure opératoire du procédé 2 (extraction liquide-liquide avec recyclage du solvant)**

| Ordre | Opération | Durée (h) |
|---|---|---|
| 1 | Chargement de 4050 kg de *Mélange réactionnel* dans *QuenchSTR* | 0,01h |
| 2 | Chauffage à 80°C de *QuenchSTR* | 2h |
| 3 | Réaction dans *QuenchSTR* | 22h |
| 4 | Vidange partielle de *QuenchSTR* par *Gas_O* | 0,01h |
| 5 | Vidange totale de *QuenchSTR* dans *Distill* | 0,01h |
| 6 | Vidange totale de *Distill* par *Waste_O* et *MAIN_O* | 5h |

A l'étape 3.3, le système détermine des paramètres de fonctionnement suivant des modèles quess (MEQ1)

Les modèles de la bibliothèque MEQ1 servent à décrire les opérations se déroulant dans les équipements (voir Table 22 et Table 23). Ils sont extrêmement similaires dans leur principe et leur structure à ceux de la bibliothèque MOP1.

Pour les opérations de réaction (opérations 3 et 5 pour le procédé 2, opération 3 pour le procédé 3), les modèles utilisés sont strictement identiques à ceux de la Table 3.

Pour l'opération 6 du procédé 3 (la distillation de l'eau et de l'acide acétique), on utilise à l'identique le modèle décrit dans la Table 10. Les autres séparations représentées sur le diagramme d'opérations du procédé 2 sont transcrites sous forme de binômes {vidange partielle + vidange totale}. Pour les vidanges partielles, les ratios de partitions entre la matière extraite et la matière restant dans l'équipement sont ceux des tables 4 à 8. Ce qui revient de fait à conserver ces ratios pour la partition des espèces entre les flux de sortie.

Le passage du diagramme d'opérations au diagramme d'équipement ne demande donc pas un gros supplément d'informations tant que l'on utilise des équipements de la bibliothèque BEQGEN et des modèles d'opérations de la bibliothèque MEQ1. Le supplément d'information consiste essentiellement à donner des durées aux opérations.

Dans les deux cas, les bilans de matières et énergies entrantes et sortantes obtenues par le calcul sont exactement identiques à ceux obtenus avec les diagrammes d'opérations (voir tables 13 à 16).

Le calcul permet également de disposer du taux de remplissage de chaque équipement susceptible de stocker un contenu ainsi que du temps total de la séquence opératoire (voir Table 24 et Table 25). Dans le cas du procédé 2, on constate que certains volumes initialement spécifiés sont à changer afin d'aboutir à des taux d'occupation raisonnables. La méthodologie ici exposée permet un tel processus itératif.

**Table 24 : éléments de résultat pour la simulation du diagramme d'équipement du procédé 2**

| Donnée | Valeur | Volume | |
|---|---|---|---|
| | | Ancien | Nouveau |
| Taux d'occupation de *QuenchSTR* | 67% | 14 m$^3$ | 12 m$^3$ |
| Taux d'occupation de *LL_extractor* | 94% | 18 m$^3$ | 22 m$^3$ |
| Taux d'occupation de *SaltsFilter* | 82% | 10 m$^3$ | 10 m$^3$ |
| Taux d'occupation de *Evaporator* | 93% | 8 m$^3$ | 10 m$^3$ |
| Taux d'occupation de *SolvPurif* | 102% | 6 m$^3$ | 8 m$^3$ |
| Taux d'occupation de *SolventTank* | 97% | 6 m$^3$ | 8 m$^3$ |
| Temps Total | 14,1 h | | |

**Table 25 : éléments de résultat pour la simulation du diagramme d'équipement du procédé 3**

| Donnée | Valeur |
|---|---|
| Taux d'occupation de *QuenchSTR* | 67,5% |
| Taux d'occupation de *Distill* | 66,4% |
| Temps total | 29,03 h |

A ce stade, nous disposons des éléments d'information nécessaires pour procéder à un calcul de coûts fixes.

Les hypothèses relatives aux coûts variables restent d'actualité. Celles relatives aux coûts fixes sont exposées ci-dessous.

Pour le calcul du coût d'investissement d'un équipement, on se réfère à une taille et à un prix de référence selon la formule suivante :

$$\text{Prix de l'équipement (k€)} = \text{Prix de référence (k€)} \left(\frac{\text{taille}}{\text{Taille de référence}}\right)^{\text{élasticité}} \quad (1)$$

Le détail des CAPEX des deux procédés étudiés apparaît dans la Table 26 et la Table 27.

On considère que le coût de l'équipement est amorti sur 64 000 heures. Nous considérons également un coût de maintenance de 5% du CAPEX par année de 8 000 heures.

Dans les deux cas, on considère un temps d'arrêt du procédé de 2,4 heures à chaque cycle. Lorsque le procédé est en fonctionnement, on considère que chaque équipement (excepté le stockage de solvant) mobilise un équivalent temps plein coutant 1 000 €/jour.

On considère un coût de contrôle de qualité de 100 000 €/an et un coût de frais généraux de 25% des autres coûts fixes.

**Table 26 : taille, paramètres économiques et coût de chaque équipement pour le procédé 2**

| Equipement | Taille | Taille de ref. | Prix de ref. (k€) | Elasticité | Prix de l'équipement (k€) |
|---|---|---|---|---|---|
| *QuenchSTR* | 12 m$^3$ | 3 m$^3$ | 1 000 | | 2 297 |
| *LL_extractor* | 22 m$^3$ | | | | 4 958 |
| *SaltsFilter* | 10 m$^3$ | 3 m$^3$ | 1 500 | 0.6 | 3 089 |
| *Evaporator* | 10 m$^3$ | | | | 3 089 |
| *SolvPurif* | 8 m$^3$ | | | | 2 702 |
| *SolventTank* | 8 m$^3$ | 3 m$^3$ | 100 | | 180 |
| Total CAPEX | | | | | 16 315 |

**Table 27 : taille, paramètres économiques et coût de chaque équipement pour le procédé 2**

| Equipement | Taille | Taille de ref. | Prix de ref. (k€) | Elasticité | Prix de l'équipement (k€) |
|---|---|---|---|---|---|
| *QuenchSTR* | 6 m$^3$ | 3 m$^3$ | 1 000 | 0.6 | 1 516 |
| *Distill* | 6 m$^3$ | 3 m$^3$ | 1 500 | | 2 274 |
| Total CAPEX | | | | | 3 789 |

Il y a lieu de signaler que, dans la continuité du recours aux séparateurs génériques, on fait l'hypothèse que tous ces séparateurs ont les mêmes paramètres de coûts d'investissement. Il s'agit évidemment d'une hypothèse forte mais elle nous permet à ce stade une économie de temps et d'informations très importante.

**Table 28 : coût total de production (en € par kg de DVL dans *MAIN_O*) avec ventilation des coûts fixes**

| Poste | Procédé 2 | Procédé 3 |
|---|---|---|
| Amortissement CAPEX | 4.13 | 1.83 |
| Maintenance | 1.65 | 0.73 |
| Main d'oeuvre | 2.89 | 2.38 |
| Contrôle qualité | 0.20 | 0.39 |
| Frais généraux | 2.22 | 1.33 |
| Sous-total coûts fixes | 11.09 | 6.65 |
| Sous-total coûts variables | 31.94 | 28.61 |
| Total général | 43.03 | 35.26 |

Nous pouvons constater que les coûts fixes amplifient l'avantage du procédé 3 (basé sur la neutralisation thermique du peroxyde) par rapport au procédé 2 (basé sur la neutralisation saline). C'est donc sur le procédé 3 uniquement qu'il est judicieux de focaliser la suite des investigations.

Concernant le choix de recourir aux séparateurs génériques, on peut constater que, même si les contributions liées à l'équipement étaient réduites à 0 €/kg dans le cas du procédé 2, le procédé 3 resterait plus avantageux. Le choix des séparateurs génériques et l'hypothèse associée n'ont donc pas faussé la conclusion comparative.

Si ce choix n'avait pas été fait, il aurait fallu fournir une grosse quantité d'informations sur les séparateurs du procédé 2 pour ensuite se rendre compte que ce procédé était à abandonner.

À ce stade de l'étude, nous n'avons eu recours à aucun paramètre cinétique et extrêmement peu de données thermodynamiques. Par ailleurs, seule la séparation par neutralisation thermique du peroxyde et distillation de l'acide acétique est encore considérée.

En d'autres termes, du point de vue des paramètres liés aux espèces, on peut dire la chose suivante :

- une approche ascendante (« bottom-up ») aurait nécessité d'emblée de collecter d'abondantes données sur chacune des 12 espèces de la Table 1. Ainsi que sur la façon dont elles peuvent interagir.

- l'approche descendante (« top-down ») ici exposée nous permet de réduire la liste d'espèces à 6 entrées (dont aucune espèce saline) avant toute collecte de données physico-chimiques, excepté des masses molaires et températures normales d'ébullition.

De la même façon, l'approche montante aurait nécessité une étude des réactions de neutralisation du peroxyde par le sulfite et de neutralisation de l'acide acétique par le carbonate. L'approche descendante, rend cette étude inutile.

Les modèles BOP1 et MEQ1 utilisés jusqu'ici nous ont permis un tri entre les configurations moyennant un investissement modeste en termes de temps et d'argent. Toutefois, pour la configuration qui apparaît comme la meilleure, ces modèles ne remplacent pas une étude avec des modèles conventionnels de génie chimique (bibliothèque MEQ2).

A ce stade, nous avons tiré le maximum de la méthode GUESS. Il est maintenant temps de s'intéresser aux points faibles ou aux imprécisions qui peuvent impacter les performances du procédé.

Lors de l'étape 3.4, on procède à la détermination par le système de paramètres de fonctionnement suivant un modèle standard (MEQ2).

La prochaine étape de l'étude amènerait trois axes de perfectionnement :

- une description de la cinétique de la neutralisation thermique du peroxyde, ce qui permettra de statuer sur la durée nécessaire pour cette réaction

- le choix d'un équipement de distillation, ce qui permettra d'évaluer plus finement les coûts d'investissement

- une description de la distillation par des lois d'équilibre thermodynamique, ce qui permettra d'évaluer plus finement la qualité de la séparation.

Lors de l'étape 3.5, il est procédé à la détermination par le système de paramètres de fonctionnement suivant un modèle détaillé (MEQ3).

Il serait possible de raffiner encore l'étude en recourant à des modèles de la bibliothèque MEQ3. Ces modèles nécessitent de s'appuyer sur des équipements de la bibliothèque BEQSPEC car ils font appel à la description géométrique des équipements afin de statuer par exemple sur des non-idéalités de mélange ou la formation de points chauds.

**Exemple 2**

Un deuxième exemple d'utilisation d'un système selon des modes de réalisation est donné ci-après, en référence au procédé décrit dans le brevet US 6 444 854.

L'objectif général est de minimiser le coût de production de sertraline-tétralone énantiomériquement pure ou optiquement enrichie (en forme R) à partir d'un mélange contenant deux énantiomères (R et S) au moyen d'une chromatographie telle que la chromatographie sur lit mobile simulé (LMS)

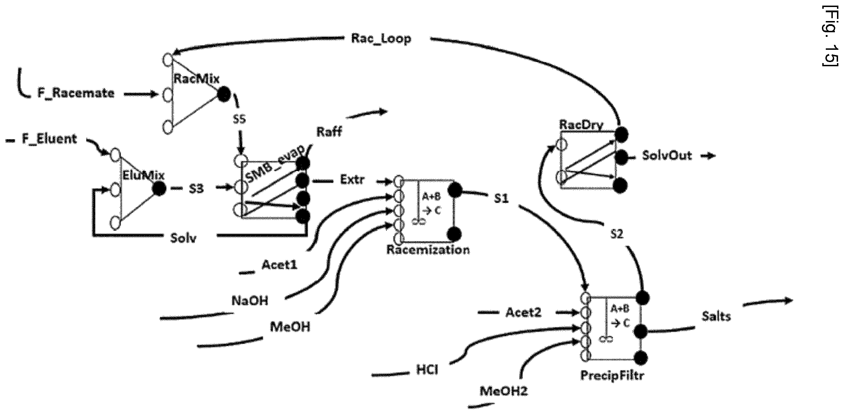

Cet objectif général est décliné en une série de questions : est-il rentable de recycler les énantiomères indésirables dans un réacteur de racémisation pour mélange du racémate ainsi obtenu avec le produit d'injection frais (voir figure 15)? Quels éléments du processus nécessitent une étude avancée pour assurer une bonne estimation des coûts et ensuite des choix techniques pertinents ? En d'autres termes, où sont les « goulots d'étranglement » en matière d'information ?

Les seules informations disponibles sur le procédé étudié proviennent du brevet précité. Ce document ne fournit aucune information fondamentale sur : la thermodynamique générale, les équilibres de sorption, la cinétique de la réaction de racémisation.

Les informations disponibles consistent en une courte série de rapports d'expériences indiquant ce qui a été injecté dans quel équipement et fournissent quelques informations macroscopiques sur les conséquences observées. Les informations contenues dans ce document pourraient aussi contenir certaines erreurs et doivent être traitées avec précautions.

Une approche classique ascendante (« bottom-up ») d'optimisation nécessiterait une longue et coûteuse campagne d'expériences pour collecter les informations fondamentales manquantes (thermodynamique, sorption, cinétique de la réaction de racémisation en particulier) pour la description de la chromatographie LMS et du réacteur de racémisation à travers des modèles classiques d'ingénierie des procédés.

Cette approche serait beaucoup trop complexe, longue et coûteuse à mettre en place dans le cadre d'une première évaluation.

Nous appliquons ci-après une approche descendante (« top-down ») suivant l'invention. Pour illustration, cette approche est utilisée pour comparer deux exemples de schéma de procédé :

- un schéma sans recyclage de l'énantiomère S, et sans racémisation,

- un schéma simple avec recyclage de l'énantiomère S, et avec racémisation.

Dans la première étape, pour la Etape 1: définition des matières premières et des espèces

Pour le schéma sans recyclage, les seules matières premières sont le mélange racémique et le solvant frais. En cas de racémisation, il convient d'ajouter de l'acétonitrile, du méthanol, de la soude et de l'acide chlorhydrique.

Au final, les matières premières utilisées et les espèces associées sont listées dans la Table 29. A ces 8 espèces injectées, s'ajoute le NaCl qui peut être généré dans le procédé

**Table 29 : matières premières et espèces associées**

| Matières premières | Espèces associées |
|---|---|
| Sertraline-tétralone racémate | R et S |

(suite)

| Matières premières | Espèces associées |
|---|---|
| Solvant frais | 10 % MeOH et 90 % CH3CN |
| Acétonitrile | CH3CN |
| Méthanol | MeOH |
| NaOH | Na+ ; OH- |
| HCl | H+ ; Cl- |
| **Soit 6 matières premières** | **Soit 8 espèces** |

Dans la deuxième étape de définition des schémas blocs d'opération et dans le cadre de l'approche descendante proposée, la représentation des procédés commence par l'établissement de diagrammes d'opérations (étape 2.1).

Les deux schémas d'opération considérés sont représentés.

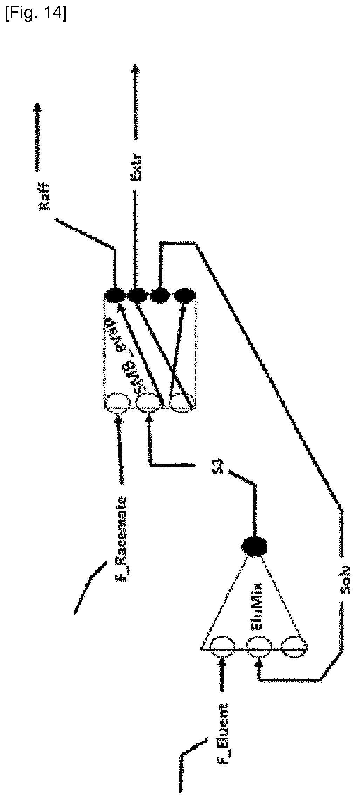

Sur la figure 14 : schéma simple, sans recyclage de l'énantiomère S, et sans racémisation.

Sur la figure 15 : schéma avec recyclage de l'énantiomère S, et avec racémisation.

Les schémas de blocs d'opération peuvent aussi bien se lire en flux (kg/h) qu'en quantité (kg) traitée par opération.

Lors de l'étape 2.2, les relations entre les entrées et les sorties des blocs d'opération sont définies.

Les données du brevet US 6 444 854 B1 et quelques hypothèses raisonnables, nous permettent de décrire le comportement de chaque opération des figures 14 et 15. Chaque opération est décrite par son effet sur les flux (ou les quantités).

Le schéma de blocs d'opération de la figure 14 ne contient que deux blocs d'opération : un bloc d'opération de mélange *EluMix et* un bloc d'opération de séparation-évaporation *SMB_Evap* qui permet de séparer les énantiomères et de les concentrer.

Le schéma de blocs d'opération de la figure 15 contient les opérations de la figure 14 et en outre, les blocs suivants.

Un autre bloc d'opération de mélange *RacMix.*

Un bloc d'opération de réaction racémisation *Racemization :* il convertit l'énantiomère-S en énantiomère-R. La transformation s'arrête lorsqu'un mélange racémique est obtenu.

Un bloc d'opération de *PrecipFiltr.*

Un bloc d'opération de séchage du racémate *RacDry.*

Nous allons désormais détailler le fonctionnement de ces différentes opérations en termes des relations entre les entrées et les sorties des blocs d'opération.

Les mélangeurs *EluMix* et *RacMix* reçoivent les matériaux d'entrée à 25°C.

Les compositions et température de sorties sont une simple combinaison linéaire de celles d'entrées ; les mélanges sont ici des liquides supposés idéaux.

Pour les opérations de séparation, les ratios de séparation décrivent comment un composé contenu dans les flux d'entrée est réparti entre les flux de sortie. La température, l'état, les rapports de fractionnement sont donnés (possiblement devinés/intuités/souhaités) par l'utilisateur. Ils sont soit de 0% ou 100% dans les tableaux ci-dessous, mais peuvent prendre n'importe quelle valeur comprise entre 0% et 100% (voir Table 33). Les valeurs sont basées sur l'expérience ou l'intuition de chimiste ou par quelques résultats du brevet US 6 444 854 B1.

Le bloc d'opération *PrecipFiltr* convertit Na+ et Cl- (dissous) en NaCl solide qui est envoyé à *SaltsOut.* (voir Table 30) :

Table 30: ratios de séparation de l'opération *PrecipFiltr*

|  | SaltsOut | S2 |
|---|---|---|
| NaCl (Solide) | 100% | 0% |
| Autres espèces | 0% | 100% |

Le bloc d'opération *RacDry* est décrit avec les paramètres de la Table 31. Ils décrivent une opération de séchage du racémate parfaite. Pour cette séparation, l'ensemble de la charge est porté à 85°C avec évaporation de *SolvOut* (enthaplie de changement d'état de 850 kJ/ka) puis les deux sorties sont ramenées à 25°C à l'état liquide.

Table 31: ratios de séparation du bloc d'opération *RacDry*

|  | SolvOut | Rac_Loop |
|---|---|---|
| R-Sertraline-tétralone | 0% | 100% |
| S-Sertraline-tétralone | 0% | 100% |
| Autres espèces | 100% | 0% |

La performance de la séparation LMS peut être calculée à partir des résultats expérimentaux exposés dans le brevet. Nous utilisons les données de la troisième expérience (voir Table 32).

Table 32: Performances LMS rapportées au cours de la troisième expérience (brevet US 6 444 854).

| Pureté de l'énantiomère le moins retenu (%) | 99.7 |
|---|---|
| Rendement de récupération de l'énantiomère le moins retenu (%) | 98,4 |
| Volume calculé d'éluent nécessaire (L/g d'énantiomères) | 0,40 |

À partir de cette information, nous pouvons calculer les ratios de séparation de l'opération *SMB_evap* (voir Table 33). Nous considérons que l'opération *SMB_evap* comprend le processus chromatographique, mais aussi d'évaporation pour enlever une grande partie des solvants. Ces solvants évaporés sont recyclés pour raisons d'économie. Pour ce faire, toute la charge de l'opération est portée à 85°C, ce qui vaporise le flux *Solv* (enthalpie de changement d'état de 850 kJ/kg). Ensuite, les trois flux de sortie sont ramenés à 25°C à l'état liquide.

Table 33: ratios de séparation du bloc d'opération *SMB_evap* dans le cas de base

|  | Raffinat | Extrait | Solvant |
|---|---|---|---|
| R-Sertraline-tétralone | 98,4% | 1,6% | 0% |
| S-Sertraline-tétralone | 0,12% | 99,88% | 0% |
| Méthanol | 0,5% | 0,5% | 99% |
| Acétonitrile | 0,5% | 0,5% | 99% |

Lors de l'étape 2.3, les bilans globaux sont déterminés. Les flux d'entrée sont dimensionnés pour obtenir un débit de 12,5 kg/h (soit 100 tonnes par an pour 8 000 heures) d'énantiomère-R dans le flux *Raffinate.*
Le débit du flux *FreshEluent* est calculé de façon à ce que le flux S3 satisfasse aux conditions de la table 3.
Dans le cas sans racémisation, nous obtenons les débits d'entrée exposés dans la Table 34. La puissance thermique à apporter au procédé s'élève à 1042 kW, la puissance à retirer est de même valeur.

Table 34: description des flux sortants intrants, et internes du procédé dans le cas sans recyclage de l'énantiomère S, et sans racémisation.

|  | Flux | Matières premières | Débit / Quantités |
|---|---|---|---|
| SORTANTS | *Raffinate (contenant R-énantiomère)* |  | 32.58 kg/h (dont 12,5 kg/h de R) |
|  | *Extract* |  | 32.96 kg/h |
| INTRANTS | *FreshRacemate* | mélange racémique d'énantiomères | 25,41 kg/h |
|  | *FreshEluent* | 90/10 mélange acétonitrile - méthanol | 50,8 L/h |
| INTERNES | *S3* |  | 4013 kg/h |
|  | *Solvent* |  | 3973 kg/h |

En cas de recyclage et de racémisation de l'énantiomère S (voir voir figure 15), le flux (quantité) *FreshRacemate* est largement abaissé (voir Table 35).

Les quantités de matières premières nécessaires à l'opération *Racemization* (C2H3N_feed1, NaOH_feed, MeOH_feed, MeOH_feed2, C2H3N_feed2, HCl_feed) et les débits sont calculés sur la base de l'expérience de racémisation décrite dans le Brevet US 6 444 854 pour garder les proportions entre ces matières premières et la quantité d'énantiomères à racémiser. En particulier, le débit (quantité) d'acétonitrile à fournir est égal à 30 litres pour chaque kilogramme de S-énantiomère qui est alimenté à la racémisation. Le flux (quantité) Extr qui arrive à l'opération *Racemization* contient de l'acétonitrile qui est déduit de l'alimentation C2H3N_feed1.

Les paramètres de performance de la chromatographie en LMS (voir Table 33) ne sont pas affectés par la racémisation.

Dans ce deuxième cas, la puissance thermique à apporter au procédé s'élève à 1182 kW ; la puissance thermique à retirer s'élève à 1131 kW.

Table 35: description des intrants, sortants et internes du procédé dans le cas avec recyclage de l'énantiomère S et racémisation.

| | Flux | Matières premières | Débit/quantités |
|---|---|---|---|
| SORTANTS | *Raffinate (contenant R-énantiomère)* | | 32,65 kg/h (dont 12,5 kg/h de R) |
| | *Solv_Out* | | 323,1 kg/h |
| | *Salt_Out* | | 0,15 kg/h |
| INTRANTS | *FreshRacemate* | mélange racémique d'énantiomères | 12,52 kg/h |
| | *FreshEluent* | 90/10 mélange acétonitrile - méthanol | 51 L/h |
| | *C2H3N_feed* | acétonitrile | 106,88 L/h |
| | *NaOH_feed* | NaOH | 0,1 kg/h |
| | *MeOH_feed* | méthanol | 0,07 L/h |
| | *MeOH_feed2* | méthanol | 12,89 kg/h |
| | *C2H3N_feed 2* | acétonitrile | 257,76 L/h |
| | *HCl_feed* | HCl | 1,94 kg/h |
| INTERNES | *S3* | | 4029 kg/h |
| | S5 | | 25,42 kg/h |
| | *Extr* | | 33,04 kg/h |
| | *Solvent* | | 3989 kg/h |
| | *S1* | | 117,6 kg/h |
| | *S2* | | 336,0 kg/h |
| | *RacLoop* | | 12,90 kg/h |

Il convient de noter que les résultats de la Table 34 et de la Table 35 sont obtenus par prise en compte des relations entre les entrées et les sorties de chaque bloc d'opération et des connections entre blocs d'opération et qu'il s'agit d'un premier résultat de calcul de simulation.

Le système peut maintenant utiliser le résultat de la simulation (c'est-à-dire les flux ou quantités circulant dans les procédés donnés dans les tables 34 et 35) pour déterminer une première évaluation des coûts de productions variables. Pour l'évaluation économique, nous considérons les hypothèses suivantes qui proviennent de l'expérience. Ce sont des ordres de grandeur ;

- les prix des matières premières sont ceux énumérés dans la Table 36

- les sorties de solvants sont considérées comme déchets avec un coût de traitement de 0,15 €. Le coût du traitement des autres extrants secondaires (*Extr* pour le processus sans racémisation, *SaltsOut* pour le processus avec racémisation) est négligé.

- pour les échanges thermiques, on considère un coût de 0.10 €/kWh lorsqu'il s'agit d'énergie à apporter au procédé (chauffage/évaporation) et de 0.05 €/kWh lorsqu'il faut retirer cette énergie (refroidissement/condensation).

Table 36 : prix des matières premières

| Matières premières | Coût (€/kg) |
|---|---|
| Sertraline-tétralone racémate | 100 |
| Acétonitrile | 20 |
| Méthanol | 2 |
| 90/10 mélange acétonitrile-méthanol | 19 |
| NaOH | 0,43 |
| HCl | 0,75 |

Les informations contenues dans les tables 34 à 36 conduisent aux coûts variables des deux versions du procédé exposés dans la Table 37.

Table 37: contribution de chaque entrée à la partie variable du coût de production d'énantiomère-R. Les valeurs sont exprimées en €/kg d'énantiomère-R dans le flux *Raff*.

|  | Procédé 1 | Procédé 2 | Procédé 3 |
|---|---|---|---|
|  | Sans racémisation | Avec recyclage de l'énantiomère-S et racémisation | Avec recyclage de l'énantiomère-S, racémisation et recyclage de l'acétonitrile |
| FreshRacemate | 203, 25 | 100,12 | 100,12 |
| Total Acétonitrile (deux alimentations) | - | 461,10 | - |
| Total Méthanol (deux alimentations) | - | 2,07 | 2,07 |
| Frais éluant | 60,99 | 61,27 | 61,27 |
| NaOH_feed | - | < 0,01 | < 0,01 |
| HCl_feed | - | 0,12 | 0,12 |
| Traitement des effluents | 0,00 | 3,88 | 0,46 |
| Énergie | 12,51 | 13,58 | 15,68 |
| Total (€/kg de R dans Raffinate) | 276,75 | 642,14 | 190,53 |

Nous pouvons remarquer que le recyclage et la racémisation (procédé 2) sont fortement pénalisés par la consommation d'acétonitrile qui est utilisé comme solvant de réaction.

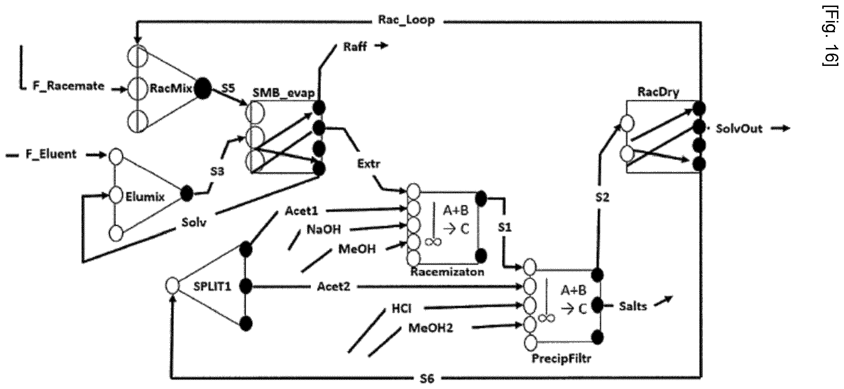

Une purification et un recyclage partiel de cet acétonitrile (voir figure 16) pourrait être considéré. Nous utilisons la méthodologie précédente pour évaluer cette troisième option. On montre qu'avec un recyclage de 94% de l'acétonitrile, aucune addition d'acétonitrile n'est nécessaire, mais que le coût de l'énergie est augmenté de 2,10 € par kg d'énantiomère-R dans *Raff*. Le coût variable total est égal à 190,53 €/kg, ce qui est plus intéressant que les autres options.

Avec cette première approche, nous avons montré que l'option 2 est très susceptible d'être peu intéressante d'un point de vue économique et en termes de production de déchets. Dans la suite, nous ne considérerons que les options 1 et 3.

La définition des schémas d'équipements est ensuite décrite en références aux diverses sous-étapes déjà évoquée ci-avant.

Dans l'étape 3.1, chacun des blocs d'opération des schémas précédents est affecté à un équipement.

La Table 38 fournit une correspondance entre les blocs d'opération et les équipements. Les diagrammes d'équipement générés sont illustrés sur les figures 9 et 10. Les équipements utilisés ici appartiennent tous à la bibliothèque BEQGEN.

**Table 38 : correspondance entre blocs d'opération et équipements. Les types d'équipements correspondent à ceux définis dans la bibliothèque BEQGEN.**

| Bloc d'opération | équipement (nom) | équipement (type) | Volume ($m^3$) |
|---|---|---|---|
| *RacMix* | *RacemateMixer* | Mélangeur | 2.5 |
| *EluMix* | *EluentMixer* | Mélangeur | 4.6 |
| *Racemisation* | *RacemisationReactor* | Réacteur | 6 |
| *PrecipFiltr* | | | |
| *Split1* | | | |
| *RacDry* | *RacemateDryer* | Séparateur | 6 |
| *SMB_evap* | *SMB_evap* | Séparateur | 0.3 |

Pour cet exemple, une opération est affectée à un équipement (Table 38), hormis pour *RacemizationReactor* qui regroupe plusieurs opérations.

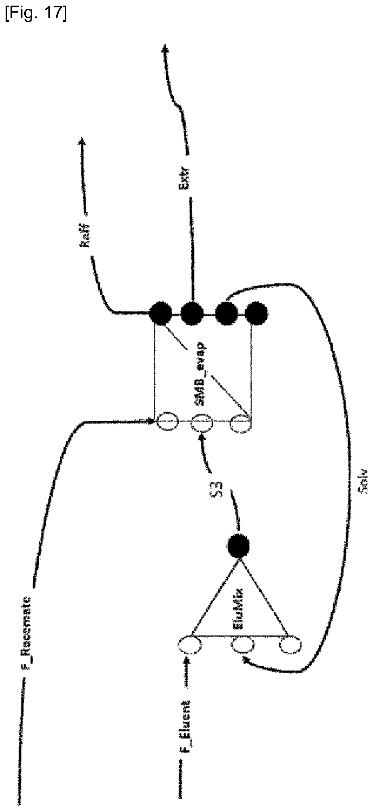

Dans le cas du diagramme de la figure 17, les deux équipements fonctionnent en continu avec les alimentations exposés dans la Table 34.

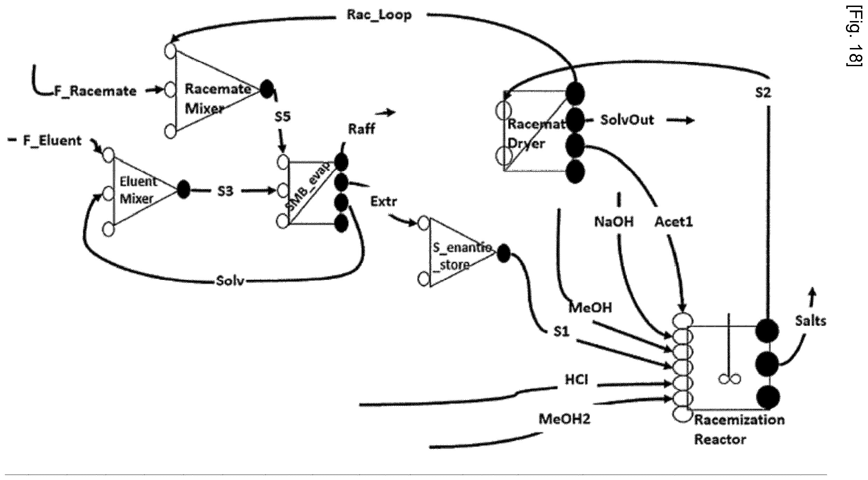

Dans le cas du diagramme de la figure 18, une partie du procédé est opérée en continu (*EluentMixer* et *SMB_evap*) là où une autre partie du procédé fonctionne de façon discontinue (*RacemizationReactor* et *RacemateDryer*) sur des cycles de 20 heures (dont 10 heures de réaction). Ces cycles suivent une procédure de la même nature que celle exposée dans la Table 22 et la Table 23 de l'exemple précédent. La procédure de ce second cas d'étude ne sera pas détaillée ici.

Les équipements *RacemateMixer* et *S_enantio_store* doivent donc être en mesure de stocker la production d'un cycle. Ce second équipement de stockage est créé par nécessité pratique, il n'a pas d'équivalent dans le diagramme d'opérations car l'approche utilisée a permis de faire jusqu'ici abstraction des contraintes d'interfaçage continu-discontinu.

Les opérations effectuées dans les équipements des deux procédés étudiés sont décrites suivant des modèles de la bibliothèque MEQ1. Ces modèles suivent les mêmes principes directeurs que ceux de la bibliothèque MOP1 (pas d'utilisation de données physico-chimiques, description macroscopique des phénomènes) ainsi que la même structure. De ce fait, les ratios de partition des tables 30 à 33, de même que la description de la réaction de racémisation, conservent toute leur validité. Les bilans de matières et d'énergies entrantes et sortantes sont donc inchangés (voir Table 34 pour le cas sans racémisation).

Contrairement aux diagrammes d'opérations, les diagrammes d'équipements gèrent les notions de temps et de taille d'équipements. Ainsi, on peut accéder à des paramètres de fonctionnement tels que le taux maximal de remplissage ou la productivité de tel ou tel équipement. Les valeurs de ces paramètres de fonctionnement permettent à l'utilisateur de juger de la pertinence pratique des tailles d'équipements et temps d'opérations qu'il a spécifié. Dans le cas présent, compte-tenu des tailles initiales données dans la Table 38, le taux d'occupation maximal du réacteur de racémisation et du sécheur de racémate est d'environ 130%, ce qui évidemment irréaliste. De même, on aboutirait pour le *SMB_evap* à une productivité de 42 kg d'énantiomère R par heure et par $m^3$, là où les valeurs courantes pour de telles séparations sont plutôt de l'ordre de 25 kg d'énantiomère R par heure et par $m^3$. Pour ce qui est de la racémisation, le document US 6,444,854 nous rapporte une expérience où 100 g d'énantiomère S sont traités en 6 heures dans un volume de 3 à 5 litres de solution. Cette réaction, telle qu'elle est menée dans le cadre de cette expérience possède donc une productivité de 3 - 4 $kg_{S, \text{entrée}}/(h.m^3_{\text{contenant}})$.

Un processus itératif permet d'ajuster les tailles des équipements de façon à ce que les paramètres de fonctionnement résultants atteignent des valeurs conformes aux attentes. On aboutit ainsi aux tailles et paramètres de fonctionnement exposés dans la Table 39.

On constate notamment que, pour le réacteur de racémisation, la contrainte sur le taux de remplissage impose de travailler à une productivité inférieure à celle autorisée par les phénomènes chimiques.

**Table 39 : dimension des principaux équipements pour une production de 12,5 kg/h d'énantiomère R ; l'éventuelle racémisation étant effectuée par cycles de 20 heures**

| Equipement | Vol. ($m^3$) | Paramètre | Valeur |
|---|---|---|---|
| *SMB_evap* | 0.5 | Productivité | 25 $kg_R/(h.m^3)$ |

(suite)

| Equipement | Vol. (m$^3$) | Paramètre | Valeur |
|---|---|---|---|
| *RacemateDryer* | 10 | Remplissage | environ 80% |
| *RacemizationReactor* | 10 | Remplissage | environ 80% |
| | | Productivité | 2,5 kgs$_{S, entrée}$/(h.m$^3_{contenant}$) |
| *RacemateMixer* | | | |
| *EluentMixer* | | Non considérés | |
| *S_enantio_store* | | | |

Nous disposons à ce stade du dimensionnement de tous les équipements essentiels à la production.

Le système peut se servir de cette information pour effectuer une première estimation économique des coûts fixes.

Les coûts des matières premières sont ceux de Table 36. Les hypothèses sur les coûts variables restent les mêmes que précédemment.

Le mode de calcul du coût des équipements est exactement le même que dans l'exemple 1 .Avec les informations données dans la Table 40 on peut donc estimer le coût des différents équipements.

Table 40 : taille, paramètres économiques et coût de chaque équipement ;

| équipement | Taille | Unité de taille | Taille de réf. | Prix de réf. (k€) | Élasticité | Coût (k€) de l'équipement |
|---|---|---|---|---|---|---|
| *SMB_evap* | 0,5 | m$^3$ | 1 | 30 000 | | 19 793 |
| *RacemizationReactor* | 10 | m$^3$ | 3 | 1 000 | 0,6 | 2 060 |
| *RacemateDryer* | 10 | m$^3$ | 3 | 1 500 | | 3 090 |
| Total CAPEX (sans racémisation ni recyclage d'acétonitrile) | | | | | | 19 793 |
| Total CAPEX (avec racémisation et recyclage d'acétonitrile) | | | | | | 24 940 |

Comme dans l'exemple 1, la contribution de l'équipement au coût de production est calculée en considérant que le coût d'investissement de l'équipement est amorti sur 64 000 heures d'utilisation. Nous considérons également un coût d'entretien qui équivaut à 5% de CAPEX par an (soit 8,000 heures).

Dans le cas présent, le coût du travail est considéré comme secondaire (pour le but d'illustration) mais pourrait être considéré. Nous considérons un coût de contrôle de la qualité de 100 000 €/an et un coût frais généraux qui équivaut 25% des autres coûts fixes. En ce qui concerne le coût de l'adsorbant utilisé pour la chromatographie en LMS, nous considérons un coût d'achat de 10 000 €/kg, une durée d'utilisation de 16 000 heures et une masse de 1 kg par kg d'énantiomère-R dans *Raff* par jour. Pour le cas impliquant la racémisation, on considère que le procédé est arrêté 10 heures entre les cycles de 20 heures.

Table 41: coût total de production (en € par kg d'énantiomère-R dans *Raff*) avec ventilation des coûts fixes

| | Option 1 | Option 3 |
|---|---|---|
| | Sans racémisation | Avec racémisation de l'énantiomère-S et recylage de l'acétonitrile |
| Amortissement de l'équipement | 24,74 | 46.79 |
| Entretien | 9,89 | 18.72 |
| Adsorbant | 15,00 | 15,00 |
| Contrôle de la qualité | 1,00 | 1,50 |
| Frais Généraux | 12,66 | 20.50 |
| Total des coûts fixes | 63,29 | 102.51 |

(suite)

|  | Option 1 | Option 3 |
|---|---|---|
|  | Sans racémisation | Avec racémisation de l'énantiomère-S et recyclage de l'acétonitrile |
| Coûts variables totaux | 276.75 | 190.53 |
| Total (€/kg de R dans Raffinate) | 340.04 | 293.04 |

En raison de la présence d'équipements additionnels, l'option 3 a des coûts fixes plus élevés que l'option 1. Toutefois, en cumulant des coûts variables et fixes, l'option 3 semble plus attractive. Dans les études suivantes, seule cette configuration sera envisagée.

Les calculs préliminaires présentés précédemment ont soulevé l'idée d'une configuration de procédé incluant la racémisation de l'énantiomère-S et le recyclage du solvant de racémisation. Ces calculs ont également indiqué que, pour l'échelle de production envisagée, cette configuration était la plus intéressante, ce qui permet d'exclure les deux autres.

L'approche proposée dans l'invention permet de tester l'impact de certains paramètres macroscopiques de performance pour déterminer ceux qui ont l'impact le plus élevé sur le coût de production. Dans le cas présent, cette étude est assez simple. En effet, la Table 39 nous indique que, même avec des phénomènes chimiques un peu plus lents ou beaucoup plus rapides qu'estimé, la taille du réacteur de racémisation ne serait pas modifiée puisque celle-ci est fixée par la contrainte du taux de remplissage. Cela n'aurait donc aucun impact sur les coûts.

A l'inverse, la performance du SMB (« Simulated Moving Bed », acronyme anglais pour « Lit Mobile Simulé ») a un impact direct sur les coûts via la consommation d'éluent, la quantité d'adsorbant ou encore la taille du SMB qui elle est directement dépendante de la productivité.

Nous pouvons immédiatement voir que les performances de *SMB_evap* a un impact beaucoup plus grand que celui de la racémisation. Il s'ensuit que, pour avoir une meilleure compréhension du processus, nous devons axer nos études sur le SMB plutôt que sur la racémisation.

En utilisant des schémas et des modèles très simples, nous avons pu simuler différentes options de procédé et converger vers une option raisonnable sans connaissance physico-chimique particulière. Nous avons identifié que le *SMB_evap* est un équipement particulièrement important au niveau économique. Si le projet a de l'intérêt, il est évident que l'on ne peut pas se contenter du niveau Guess mais que des modèles mécanistiques/prédictifs doivent être utilisés pour confirmer, préciser, voire infirmer les hypothèses du niveau Guess. Par contre, effectuer à ce stade une autre étude de la racémisation serait une dépense inutile de temps et d'argent.

Pour une étude plus avancée du *SMB_Evap,* l'utilisation de modèles mécanistiques (bibliothèque MEQ2) qui nécessitent la détermination de paramètres physico-chimiques est nécessaire. Le cas échéant, il pourrait même se révéler nécessaire d'utiliser des modèles de la bibliothèque MEQ3 qui sont capables de simuler finement le comportement d'un équipement donné. Concernant le fonctionnement des LMS, un modèle de type MC-LDF serait certainement à recommander (Chromatographie Processes: modeling, simulation and design, Roger-Marc Nicoud, Cambridge University Press, 2015).

L'approche que nous proposons nous a permis de choisir une bonne configuration sans exiger la mesure des paramètres physico-chimiques. Maintenant, pour aller plus loin, nous avons besoin de ces paramètres physico-chimiques, mais nous savons précisément lesquels et pourquoi nous devons les mesurer.

L'approche descendante de l'invention commence par des calculs modérément complexes et économes en informations. Grâce aux résultats de ces premiers calculs, nous savons quels raffinements ont priorité, quelles informations supplémentaires doivent être

L'effet technique Du système suivant l'invention est donc de permettre l'obtention d'un schéma d'équipements d'une installation industrielle, puis la réalisation et l'exploitation de celle-ci. Grâce à l'invention, ce schéma d'équipements peut être obtenu plus rapidement que dans les méthodes traditionnelles. En outre, grâce aux options d'itération, avec amélioration des éléments les plus critiques, le schéma d'équipement obtenu peut présenter des paramètres de fonctionnement meilleurs que ceux obtenus par les méthodes traditionnelles. La méthode et le dispositif de l'invention peuvent être utilisées par un chimiste de laboratoire, qui n'a pas nécessairement les compétences et l'expérience d'un ingénieur de procédé.

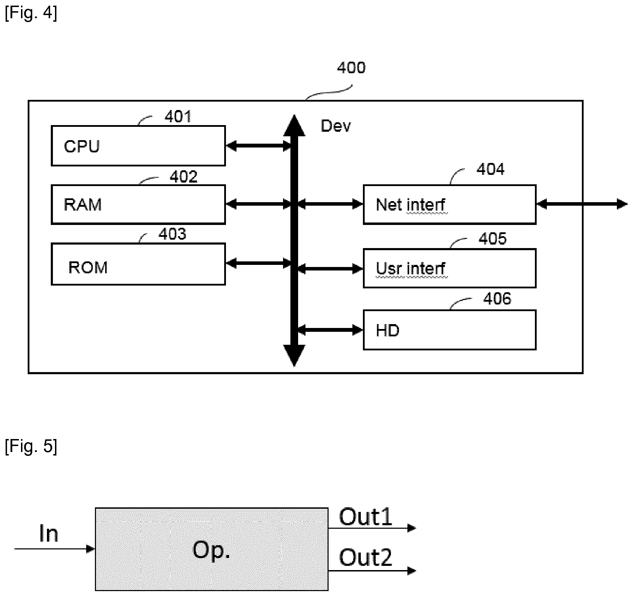

Les diverses parties du système décrites ci-dessus peuvent être mises en oeuvre par un ou plusieurs programmes d'ordinateur. Ainsi, chaque module peut correspondre à une routine d'un ou plusieurs programmes d'ordinateur. Le système est alors mis en oeuvre par un dispositif global 400 tel qu'illustré sur la **figure 4.**

Le dispositif 400 comprend un bus de communication relié à :

- une unité centrale de traitement 401 tel qu'un microprocesseur, autrement nommée CPU ;

- une mémoire à accès aléatoire 402, autrement nommée RAM, pour stocker un code exécutable du procédé des modes de réalisation de l'invention ainsi que les registres adaptés pour enregistrer les variables et les paramètres nécessaires à la mise en oeuvre du procédé conformément aux modes de réalisation, la capacité de la mémoire pouvant être augmentée par une RAM optionnelle connectée par exemple à un port d'expansion ;

- une mémoire à lecture seule 403, autrement nommée ROM, pour stocker les programmes d'ordinateur utilisés pour mettre en oeuvre les modes de réalisation de l'invention ;

- une interface de réseau 404, qui est typiquement connectée à un réseau de communication sur lequel des données numériques devant être traitées sont transmises ou reçues. L'interface de réseau 404 peut être une seule interface de réseau ou être composée d'un ensemble d'interfaces de réseau différentes (par exemple des interfaces filaires et sans-fil, ou différentes sortes d'interfaces filaires ou sans-fil). Les données sont écrites sur l'interface de réseau pour transmission ou lues à partir de l'interface réseau pour réception sous le contrôle de l'application logicielle fonctionnant dans le CPU 401 ;

- une interface utilisateur 405 pour la réception des saisies d'un utilisateur ou pour l'affichage d'information à l'utilisateur ;

- un disque dur 406 autrement nommé HD

- un module entrée/sortie 407 (autrement nommé I/O) pour envoyer/recevoir des données de/vers des dispositifs tels qu'une source vidéo ou un écran d'affichage.

Le code exécutable peut être stocké, soit dans la mémoire à lecture seule 403, soit sur le disque dur 406, ou sur un support numérique amovible tel qu'un disque par exemple. Selon une variante, le code exécutable des programmes peut être reçu au moyen d'un réseau de communication, via l'interface de réseau 404, afin d'être stocké sur un des supports de stockage du dispositif de communication 400, tel que le disque dur 406, avant d'être exécuté.

L'unité centrale de traitement 401 est adaptée pour contrôler et diriger l'exécution des instructions ou des portions de code logiciel du programme conformément aux modes de réalisation de l'invention, lesquelles instructions sont stockées sur l'un des supports de stockage précités. Après avoir été mise en service, la CPU 401 est capable d'exécuter les instructions à partir de la mémoire RAM principale 402 en rapport avec une application logicielle par exemple après que ces instructions ont été chargées à partir du programme ROM 403 ou sur le disque dur (HD) 406. Cette application logicielle, lorsqu'elle est exécutée par la CPU 401, amène les étapes de procédé à être mises en oeuvre conformément aux modes de réalisation.

**Exemple 3**

Dans ce qui suit, il est décrit un **exemple de simulation** d'une opération de réaction (Op.) avec séparation des produits en aval comme illustré sur la **figure 5.** Cette simulation est faite selon les principes décrits en détails ci-avant. La réaction est la suivante : $S1 + S3 \rightarrow S2 + S4$ avec consommation totale de S3. Les quantités d'entrée du réacteur sont données par le tableau 1.

[Tableau 1]

| Espèce | Flux entrant (In) en moles |
|--------|----------------------------|
| S1 | 10 |
| S2 | 0 |
| S3 | 5 |
| S4 | 0 |

En aval de la réaction les espèces sont réparties entre deux sorties, nommées Out1 et Out 2, selon les ratios de répartition donnés dans le tableau 2.

[Tableau 2]

| Espèce | Ratio de répartition dans le premier flux de sortie (Out1) | Ratio de répartition dans le second flux de sortie (Out2) |
|--------|--------|--------|
| S1 | 10% | 90% |
| S2 | 5% | 95% |
| S3 | 50% | 50% |
| S4 | 95% | 5% |

Les vecteurs d'états d'entrée, de première sortie et de seconde sortie contiennent les nombres de moles des différentes espèces et sont respectivement :

$$Y_E = \begin{pmatrix} m_{S1}^{In} \\ m_{S2}^{In} \\ m_{S3}^{In} \\ m_{S4}^{In} \end{pmatrix}, Y_{S1} = \begin{pmatrix} m_{S1}^{Out1} \\ m_{S2}^{Out1} \\ m_{S3}^{Out1} \\ m_{S4}^{Out1} \end{pmatrix}, Y_{S2} = \begin{pmatrix} m_{S1}^{Out2} \\ m_{S2}^{Out2} \\ m_{S3}^{Out2} \\ m_{S4}^{Out2} \end{pmatrix}$$

On obtient les équations algébriques explicites (Ex1, Ex2) suivantes :

$$(\text{Ex1}) \; A_{S1} = \begin{pmatrix} 0,1 & 0 & -0,1 & 0 \\ 0 & 0,05 & 0,05 & 0 \\ 0 & 0 & 0 & 0 \\ 0 & 0 & 0,95 & 0,95 \end{pmatrix}; \; Y_{S1} = A_{S1}Y_E$$

$$(\text{Ex2}) \; A_{S2} = \begin{pmatrix} 0,9 & 0 & -0,9 & 0 \\ 0 & 0,95 & 0,95 & 0 \\ 0 & 0 & 0 & 0 \\ 0 & 0 & 0,05 & 0,05 \end{pmatrix}; \; Y_{S2} = A_{S2}Y_E$$

Dans cet exemple, on considère que lors de la séparation les espèces sont acheminées avant leur sortie vers deux cellules parfaitement homogènes dont les nombres de moles des différentes espèces sont regroupés dans les vecteurs $\tilde{X}_1$ et $\tilde{X}_2$. On considère également que la matière soutirée des cellules par les sorties (Out1 et Out2) est de la même composition que celle des cellules. On peut alors écrire :

$$Y_{S1} = \tilde{X}_1 \text{ et } Y_{S2} = \tilde{X}_2.$$

On peut transformer le système (Ex1) (Ex2) en système différentiel en introduisant des pseudo variables d'état interne ($\tilde{X}_1$ et $\tilde{X}_2$) et en posant:

$$(\text{Ex3}) \; -\theta \frac{d\tilde{X}_1}{dt} - \tilde{X}_1 + A_{S1}Y_E = 0$$

et

$$(\text{Ex4}) \; -\theta \frac{d\tilde{X}_2}{dt} - \tilde{X}_2 + A_{S2}Y_E = 0 \; ;$$

où θ est la constante de temps arbitraire des cellules homogènes ; lorsque t devient très supérieur à θ le terme différentiel s'annule et les pseudo-variables d'état convergent vers:

$$\widetilde{X}_1 = A_{S1}Y_E \text{ et } \widetilde{X}_2 = A_{S2}Y_E$$

Pour retrouver (Ex1) et (Ex2) il suffit de poser :

$$Y_{S1} = \widetilde{X}_1 \text{ et } Y_{S2} = \widetilde{X}_2.$$

En choisissant une constante de temps faible, on passe ainsi des équations algébriques explicites (Ex1 et Ex2) à des équations différentielles (Ex3 et Ex4). Ces équations différentielles, faisant intervenir des pseudo-variables d'état interne, peuvent ensuite être introduites dans le système d'équations différentielles simulant l'intégralité du procédé chimique ou biochimique.

**Revendications**

1. Système (200, 400, 600) de simulation de procédé chimique ou biochimique, comprenant au moins une réaction ou une séparation transformant au moins une matière première, ledit système comportant :

   - une pluralité de modules fonctionnels (604, 605, 606) configurés pour réaliser des niveaux de simulation respectifs dudit procédé chimique ou biochimique, dans laquelle :

      ◦ au moins un module fonctionnel permet une simulation utilisant une modélisation algébro-différentielle basée sur une équation de conservation des espèces, et
      ◦ au moins un module fonctionnel permet une simulation utilisant une modélisation algébrique reliant des entrées et des sorties et/ou des états initiaux et des états finaux d'au moins ladite au moins une réaction,

   - au moins un module de stockage (602) pour stocker des données expérimentales relatives à des espèces chimiques dans une structure de données exploitable par au moins un module fonctionnel de ladite pluralité,
   - un module d'évaluation de performance (607) configuré pour :

      o réaliser des estimations de performances dudit procédé basées sur l'utilisation de données expérimentales issues du module de stockage et/ou de résultats de simulation obtenus par l'un des modules fonctionnels de ladite pluralité,
      o comparer au moins deux estimations de performance entre elles ou avec des résultats expérimentaux,

   - dans lequel ledit procédé est défini par un ensemble de fichiers partagés par l'ensemble des modules du système, chaque fichier comportant une description de ladite au moins une matière première et une description d'une décomposition de ladite au moins une matière première en espèces chimiques,
   - dans lequel lesdits fichiers sont les entrées et les sorties desdits modules du système, la décomposition en espèces chimiques étant conservée tout au long des traitements.

2. Système selon la revendication 1, comportant en outre un module de traitement et d'analyse de données expérimentales (603) permettant de traiter des signaux, d'effectuer des analyses statistiques et d'identifier des paramètres de modèle.

3. Système selon la revendication 1 ou 2, comportant un module de base de données (602) rassemblant des caractéristiques de physico-chimie pour les espèces chimiques, d'une part et de composition, d'origine et/ou de coût pour les matières premières, d'autre part.

4. Système selon l'une des revendications 1 à 2, comportant :

   - un module de base de données (602) rassemblant des caractéristiques de physico-chimie pour les espèces chimiques, d'une part et de composition, d'origine et/ou de coût pour les matières premières, d'autre part,
   - un module central (601) configuré pour générer un vecteur de composition représentant une décomposition de ladite au moins une matière première en une pluralité d'espèces chimiques,

- une structure de fichiers permettant aux différents modules d'avoir accès soit aux matières premières et à leur base de données, soit aux espèces et à leur base de données, soit aux deux en fonction des besoins.

**5.** Système selon l'une des revendications précédentes pour la simulation par ordinateur d'un procédé chimique ou biochimique comprenant au moins une première opération chimique ou biochimique; ledit système comprenant :

- un module de résolution (204) configuré pour la résolution d'équations algébro-diféŕentielles,
- un module de réception (201) configuré pour recevoir une première équation algébrique explicite représentant ladite première opération chimique ou biochimique et reliant un premier vecteur d'état d'entrée représentant des grandeurs chimiques initiales de ladite première opération à un premier vecteur de variables d'état de sortie représentant des grandeurs chimiques finales de ladite première opération; et
- un module de traitement (203) configuré pour :

  o dans une première équation algébro-différentielle reliant :

  • ledit premier vecteur de variables d'état d'entrée ; et
  • un premier vecteur de variables d'état interne de ladite première opération en tant qu'inconnue de l'équation;

  o injecter dans ladite première équation algébro-différentielle l'expression du vecteur de variables d'état de sortie de ladite première opération selon ladite première équation algébrique explicite en tant que vecteur de pseudo-variables d'état interne de ladite première opération, la solution en régime permanent de ladite équation algébro-différentielle ainsi obtenue convergeant ainsi vers ledit premier vecteur de variables d'état de sortie selon la première équation algébrique explicite,
  ∘ fixer une constante de temps de ladite équation algébro-différentielle ainsi obtenue comme étant inférieure à un temps caractéristique de ladite première opération, et
  o mettre en oeuvre le module de résolution sur l'équation algébro-différentielle ainsi obtenue pour calculer ledit premier vecteur de variables d'état de sortie.

**6.** Système (200) selon la revendication 5, comprenant en outre un module de sélection (202) configuré pour sélectionner un mode de simulation et dans lequel le module de traitement (203) est configuré pour réaliser ladite injection en fonction du mode sélectionné.

**7.** Système (200) selon la revendication 5 ou la revendication 6; pour la simulation par ordinateur d'un procédé chimique ou biochimique comprenant en outre une deuxième opération chimique ou biochimique ; dans lequel :

- ledit module de réception (201) est en outre configuré pour recevoir une deuxième équation algébro-différentielle représentant ladite deuxième opération chimique ou biochimique reliant :

  ∘ un deuxième vecteur de variables d'état d'entrée représentant des grandeurs chimiques initiales de ladite deuxième opération ; et
  o un deuxième vecteur de variables d'état interne de ladite deuxième opération en tant qu'inconnue de l'équation;
  la solution en régime permanent de ladite deuxième équation algébro-différentielle convergeant vers un deuxième vecteur de variables d'état de sortie représentant des grandeurs chimiques finales de ladite deuxième opération,

- ledit module de traitement (203) est en outre configuré

  o pour fusionner lesdites première et deuxième équations algébro-différentielles et
  o pour mettre en oeuvre le module de résolution (204) sur la fusion desdites première et deuxième équations algébro-différentielles ainsi obtenue pour calculer un vecteur de variables d'état de sortie du procédé.

**Patentansprüche**

**1.** System (200, 400, 600) zur Simulation eines chemischen oder biochemischen Prozesses, umfassend mindestens eine Reaktion oder eine Separation, die mindestens ein Ausgangsmaterial umwandelt, wobei das System umfasst:

- eine Vielzahl Funktionsmodule (604, 605, 606), die derart konfiguriert sind, dass sie jeweilige Simulationsstufen des chemischen bzw. biochemischen Prozesses ausführen, wobei:

  ◦ mindestens ein Funktionsmodul eine Simulation unter Verwendung einer auf einer Spezies-Erhaltungsgleichung basierenden algebraischen Differentialmodellierung ermöglicht und
  ◦ mindestens ein Funktionsmodul eine Simulation unter Verwendung einer algebraischen Modellierung, die Ein- und Ausgänge und/oder Ausgangs- und Endzustände mindestens der mindestens einen Reaktion ermöglicht,

- - mindestens ein Speichermodul (602) zum Speichern der die chemischen Spezies betreffenden Versuchsdaten in einer von mindestens einem der Vielzahl Funktionsmodule nutzbaren Datenstruktur;
- ein Leistungsbewertungsmodul (607), das konfiguriert ist zum:

  o Ausführen von Leistungsschätzungen über das Verfahren anhand der Versuchsdaten des Speichermoduls und/oder der von einem der Vielzahl Funktionsmodul erreichten Simulationsergebnisse;
  o Vergleichen mindestens zweier Leistungsschätzungen miteinander oder mit Versuchsergebnissen,

- wobei das Verfahren definiert wird durch eine Gruppe von Dateien, die von allen Modulen des Systems geteilt werden, wobei jede Datei eine Beschreibung des mindestens einen Ausgangsmaterials und eine Beschreibung eines Abbaus des mindestens einen Ausgangsmaterials in chemische Spezies enthält,
- wobei es sich bei den Dateien um die Ein- und Ausgänge der Module des Systems handelt, wobei der Abbau in chemische Spezies über den ganzen Bearbeitungsverlauf erhalten bleibt.

2. System nach Anspruch 1, ferner umfassend ein Modul (603) zur Bearbeitung und Auswertung von Versuchsdaten, die die Bearbeitung von Signalen, die Ausführung statistischer Auswertungen und die Identifizierung von Modellparametern ermöglicht.

3. System nach Anspruch 1 oder 2, umfassend ein Datenkbanmodul (602), in dem einerseits die physikalisch-chemischen Eigenschaften der chemischen Spezies und andererseits Zusammensetzung, Herkunft und/oder Kosten der Ausgangsmaterialien zusammengetragen werden.

4. System nach einem der Ansprüche 1 - 2, umfassend:

  - ein Datenkbanmodul (602), in dem einerseits die physikalisch-chemischen Eigenschaften der chemischen Spezies und andererseits Zusammensetzung, Herkunft und/oder Kosten der Ausgangsmaterialien zusammengetragen werden.
  - ein zentrales Modul (601), das zur Erzeugung eines einen Abbau des mindestens einen Ausgangsmaterials in eine Vielzahl chemischer Spezies darstellenden Zusammensetzungsvektors konfiguriert ist;
  - eine Dateistruktur, die den verschiedenen Modulen nach Bedarf den Zugriff sowohl auf die Ausgangsmaterialien und deren Datenbank als auch auf die Spezies und deren Datenbunk sowie auf beide gewährt.

5. System nach einem der vorstehenden Ansprüche zum rechnergestützten Simulieren eines chemischen oder biochemischen Verfahrens, umfassend mindestens einen ersten chemischen bzw. biochemischen Vorgang, wobei das System umfasst:

  - ein Lösungsmodul (204), das zum Lösen algebraischer Differentialgleichungen konfiguriert ist,
  - ein Empfangsmodul (201), das derart konfiguriert ist, dass es eine den ersten chemischen bzw. biochemischen Vorgang darstellende erste ausdrückliche algebraische Gleichung, die einen chemische Ausgangsgrößen des ersten Vorgangs darstellenden Ausgangszustandsvektor mit einem die letztendlichen chemischen Größen des ersten Vorgangs darstellenden ersten Endzustandsvariablenvektor verbindet, empfängt, und
  - ein Bearbeitungsmodul (203), das derart konfiguriert ist, dass es

    o in einer ersten algebraischen Differentialgleichung zur Verbindung

      • des ersten Ausgangszustandsvariablen Vektors mit
      • einem ersten Vektor von Variablen, die den internen Zustand des ersten Vorgangs betreffen, als unbekannter Größe,

o in die erste algebraische Differentialgleichung den Ausdruck des Endzustandsvariablenvektors des ersten Vorgangs gemäß der ersten ausdrücklichen algebraischen Gleichung als Vektor von Pseudovariablen des internen Zustands des ersten Vorgangs einführt, wobei die derart ermittelte Lösung der algebraischen Differentialgleichung im stationären Zustand in Richtung des ersten Endzustandsvariablenvektors gemäß der ersten ausdrücklichen algebraischen Gleichung konvergiert,
o eine Zeitkonstante der derart ermittelte algebraischen Differentialgleichung unterhalb einer charakteristischen Zeit des ersten Vorgangs festsetzt und
∘ das Lösungsmodul die derart ermittelte algebraische Differentialgleichung bearbeiten lässt, um den ersten Endzustandvariablenvektor zu errechnen.

6. System (200) nach Anspruch 5, ferner umfassend ein Auswahlmodul (202), das derart konfiguriert ist, dass es einen Simulationsmodus auswählt, wobei das Bearbeitungsmodul (203) zur Ausführung der Einführung als Funktion des ausgewählten Moduls konfiguriert ist.

7. System (200) nach Anspruch 5 oder 6 zur rechnergestützten Simulation eines chemischen oder biochemischen Vorgangs, ferner umfassend einen zweiten chemischen bzw. biochemischen Vorgang, wobei

- das Empfangsmodul (201) ferner derart konfiguriert ist, dass es eine zweite algebraische Differentialgleichung empfängt, die den zweiten chemischen bzw. biochemischen Vorgang darstellt, welche

o einen zweiten Ausgangszustandsvariablenvektor, der chemische Ausgangsgrößen des zweiten Vorgangsdarstellt mit
∘ einem ersten Vektor von Variablen, die den internen Zustand des ersten Vorgangs betreffen, als unbekannter Größe, verbindet,

wobei die Lösung der zweiten algebraischen Differentialgleichung im stationären Zustand in Richtung eines zweiten Endzustandsvariablenvektor, der chemische Endgrößen des zweiten Vorgangs darstellt, konvergiert,
- wobei das Bearbeitungsmodul (203) ferner konfiguriert ist zum

o Fusionieren der ersten und zweiten algebraischen Differentialgleichungen und
o Ausführen des Lösungsmoduls (204) an der Fusion der derart ermittelten ersten und zweiten algebraischen Differentialzleichung, um einen Ausgangs- und Endvariablenvektor des Vorgangs zu errechnen.

**Claims**

1. A system (200, 400, 600) for simulating a chemical or biochemical process, comprising at least one reaction or one separation transforming at least one raw material, said system comprising:

- a plurality of functional modules (604, 605, 606) configured to carry out respective levels of simulation of said chemical or biochemical process, wherein:

∘ at least one functional module allows a simulation using a differential-algebraic modelling based on an equation of conservation of species, and
∘ at least one functional module allows a simulation using an algebraic modelling, linking together inputs and outputs, and/or initial states and end states of at least said at least one reaction,

- at least one storage module (602) for storing experimental data relating to chemical species in a data structure able to be used by at least one functional module of said plurality,
- a performance evaluation module (607) configured:

∘ to carry out performance estimations of said process based on the use of experimental data derived from the storage module and/or from simulation results obtained by one of the functional modules of said plurality,
∘ to compare at least two performance estimations with each other or with experimental results,

- wherein said process is defined by a set of files shared by all the modules of the system, each file comprising a description of said at least one raw material and a description of a decomposition of said at least one raw material into chemical species,

- wherein said files are the inputs and outputs of said modules of the system, the decomposition into chemical species being preserved throughout all processing.

2. The system according to claim 1, further comprising a processing and analysis module (603) of experimental data, allowing processing of signals, performing of statistical analyses and identification of model parameters.

3. The system according to claim 1 or 2, comprising a database module (602) grouping together physicochemical characteristics for the chemical species, on the one hand, and of composition, origin and/or cost for the raw materials, on the other hand.

4. The system according to one of claims 1 to 2 comprising:

   - a database module (602) grouping together physicochemical characteristics for the chemical species, on the one hand, and of composition, origin and/or cost for the raw materials, on the other hand,
   - a central module (601) configured to generate a composition vector representing a decomposition of said at least one raw material into a plurality of chemical species,
   - a file structure enabling the different modules to have access either to the raw materials and their database, or to the species and their database, or to both according to needs.

5. The system according to one of the preceding claims for computer simulation of a chemical or biochemical process comprising at least a first chemical or biochemical operation, said system comprising:

   - a solver module (204) configured for solving of differential-algebraic equations,
   - a receiver module (201) configured to receive a first explicit algebraic equation representing said first chemical or biochemical operation, and linking a first input state vector representing initial chemical amounts of said first operation with a first vector of output state variables representing final chemical amounts of said first operation; and
   - a processing module (203) configured:

     ◦ in a first differential-algebraic equation linking:

       ▪ said first vector of input state variables; and
       ▪ a first vector of internal state variables of said first operation as unknown of the equation,

     ◦ to inject into said first differential-algebraic equation the expression of the vector of output state variables of said first operation according to said first explicit algebraic equation as vector of internal state pseudo-variables of said first operation, the steady state solution of said differential-algebraic equation thus obtained thereby converging towards said first vector of output state variables according to the first explicit algebraic equation,
     ◦ to set a time constant of said differential-algebraic equation thus obtained as being less than a characteristic time of said first operation, and
     ◦ to transmit to the solver module the differential-algebraic equation thus obtained, to calculate said first vector of output state variables.

6. The system (200) according to claim 5 further comprising a selection module (202) configured to select a simulation mode, and wherein the processing module (203) is configured to carry out said injection as a function of the selected mode.

7. The system (200) according to claim 5 or claim 6, for computer simulation of a chemical or biochemical process also comprising a second chemical or biochemical operation; wherein:

   - said receiver module (201) is also configured to receive a second differential-algebraic equation representing said second chemical or biochemical operation, linking:

     ◦ a second vector of input state variables representing initial chemical amounts of said second operation; and
     ◦ a second vector of internal state variables of said second operation as unknown of the equation;

   the steady state solution of said second differential-algebraic equation converging towards a second vector of output state variables representing final chemical amounts of said second operation,
- said processing module (203) is also configured:

　　∘ to merge said first and second differential-algebraic equations, and
　　∘ to transmit the merging of said first and second differential-algebraic equations thus obtained to the solver module (204), to calculate a vector of output state variables of the process.

[Fig. 1]

[Fig. 2]

[Fig. 3]

[Fig. 4]

400

Dev

401
CPU

402
RAM

403
ROM

404
Net interf

405
Usr interf

406
HD

[Fig. 5]

In

Op.

Out1

Out2

[Fig. 6]

[Fig. 7]

EP 3 786 733 B1

[Fig. 8]

[Fig. 9]

[Fig. 10]

EP 3 786 733 B1

[Fig. 11]

[Fig. 12]

[Fig. 13]

[Fig. 14]

[Fig. 15]

[Fig. 16]

[Fig. 17]

[Fig. 18]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2005267723 A1
- US 6444854 B
- US 6444854 B1

**Littérature non-brevet citée dans la description**

- Chromatographie Processes: modeling, simulation and design. Roger-Marc Nicoud. Cambridge University Press, 2015